# EUROPEAN PATENT APPLICATION

(11) **EP 4 834 329 A1**
(43) Date of publication of application: **04.11.2026**
(21) Application number: 26197289.7
(22) Date of filing: 08.06.2022
(51) Int. Cl.: A61K 9/14, A61K 9/16, A61K 9/20, A61K 9/48, A61K 31/506, A61P 3/00, A61P 43/00

(54) **FORMULATION CONTAINING DIHYDROPYRIDAZINE-3,5-DIONE DERIVATIVE**

(30) Priority: 08.06.2021 JP 2021096206
(62) Document number(s) of the earlier application(s) in accordance with Art. 76 EPC: 22820243.8 / 4 353 233
(71) Applicant: CHUGAI SEIYAKU KABUSHIKI KAISHA, Tokyo 115-8543 (JP)
(72) Inventor: SAKAI, Kenichi, Tokyo 115-8543 (JP); UCHIYAMA, Hiromasa, Tokyo 115-8543 (JP); MINODA, Tomoyuki, Tokyo 115-8543 (JP); YOSHIKAWA, Mayumi, Tokyo 115-8543 (JP); NATSUMOTO, Shigemori, Tokyo 115-8543 (JP); TAKANO, Ryusuke, Shizuoka 412-8513 (JP)
(74) Representative: Vossius & Partner Patentanwälte Rechtsanwälte mbB

(57) **Abstract**

The present invention provides a pharmaceutical composition having excellent dissolution property and content uniformity and a production method thereof by mixing a p-toluenesulfonate of 7-[[2,3-difluoro-4-[2-[2-methoxyethyl(methyl)amino]ethoxy]phenyl]methyl]-10-hydroxy-6-methyl-8-oxo-N-[4-(trifluoromethyl)-2-[6-(trifluoromethyl)pyrimidin-4-yl]phenyl]-6,7-diazaspiro[4,5]deca-9-ene-9-carboxamide with specific additives, and formulating the mixture.

## Description

### TECHNICAL FIELD

The present invention relates to a formulation containing a dihydropyridazine-3,5-dione derivative.

### BACKGROUND ART

Patients having kidney dysfunction such as chronic kidney disease (CKD) and end-stage kidney disease (ESKD) are known to develop hyperphosphatemia as phosphorus is accumulated in the body. Calcification of the blood vessel due to hyperphosphatemia may be a cause of dysfunction of the cardiovascular system. In addition, the calcification of the blood vessel leads to excessive secretion of parathyroid hormone, and causes a bone lesion. Thus, hyperphosphatemia may be a factor of worsening the prognosis and QOL of end-stage kidney-failure patients and dialysis patients (Non Patent Literature 1).

CKD is classified into stages 1 to 5 according to the degree of progression (Non Patent Literatures 2 and 3). The blood phosphorus level in patients with stages 3 and 4 of CKD is related to the prevalence rate and the mortality rate of cardiovascular diseases, and control of the blood phosphorus level in these patients may lead to alleviation or prevention of cardiovascular diseases. Further, earlier control of a load of phosphates on a patient may alleviate and/or prevent progression of a disease condition in a patient with early-stage CDK (Non Patent Literature 4).

For current treatment of hyperphosphatemia, phosphorus adsorbents aimed at suppressing absorption of phosphoric acid in the gastrointestinal tract are used. As the phosphorus adsorbent, non-metallic polymer adsorbents typified by sevelamer hydrochloride, calcium salt preparations typified by precipitated calcium carbonate, and metallic adsorbents typified by lanthanum carbonate are used, and poor medication compliance due to necessity of dosing at several grams a day and side effects caused by accumulation of calcium in the body have been reported. Thus, there is strong demand for development of a novel therapeutic means for hyperphosphatemia in which the above-mentioned problems of phosphorus adsorbents are improved (Non Patent Literature 4).

As sodium-dependent phosphate transporters, three families: NaPi-I, NaPi-II and NaPi-III are known. These families are further classified into isotypes, and for the NaPi-II family, NaPi-IIa, NaPi-IIb and NaPi-IIc are known. In particular, phosphate transporters such as NaPi-IIb, PiT-1 and PiT-2 are known to perform phosphorus absorption in the gastrointestinal tract, and selective inhibition of only these phosphate transporters involved in phosphorus absorption can be expected to produce a strong effect of inhibiting phosphorus absorption in the gastrointestinal tract, resulting in the reduction of the blood phosphorus level (Non Patent Literatures 5 to 8).

NTX 1942 (Patent Literature 1) and condensed thiophene derivatives (Patent Literatures 2 to 5) have been heretofore reported as NaPi-IIb inhibitors. Dihydropyridazine-3,5-dione derivatives typified by 7-[[2,3-difluoro-4-[2-[2-methoxyethyl(methyl)amino]ethoxy]phenyl]methyl]-10-hydroxy-6-methyl-8-oxo-N-[4-(trifluoromethyl)-2-[6-(trifluoromethyl)pyrimidin-4-yl]phenyl]-6,7-diazaspiro[4,5]deca-9-ene-9-carboxamide of formula 1 have been reported to have inhibitory actions on NaPi-IIb, PiT-1 and PiT-2 (Patent Literatures 6 and 7, and Non Patent Literature 9).

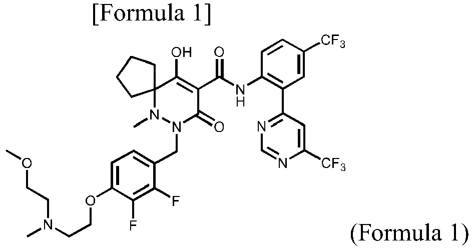

When treating a chronic disease such as hyperphosphatemia, a pharmaceutical formulation having an orally administrable dosage form that can be administered by the patient oneself is more preferable than an injection formulation that requires a hospital visit each time the dosage is administered. For the dosage form of the pharmaceutical formulations, especially when the active ingredient is contained as a solid, solid formulations are preferably used. Examples of the solid formulations include a powdered formulation, a powder, a granule, a tablet, and a capsule. When these solid formulations are provided, it is important that the active ingredient is effectively absorbed in the gastrointestinal tract after the formulation has disintegrated in the gastrointestinal tract. For example, in the capsule formulation among dosage forms of the formulations, the active ingredient (drug substance) encapsulated in a small space is administered, and, after the capsule shell has disintegrated in the gastrointestinal tract, the drug substance is dispersed following the dispersion of the filler in the capsule shell. In addition, it is preferred that the drug substance used in the pharmaceutical formulations uses drug substance particles that are optimized, because the drug substance having an optimized particle shape or particle size are appropriate in surface area of the drug substance from the viewpoint of solubility and the drug substance can dissolve efficiently and be easily absorbed from the gastrointestinal tract. Furthermore, the drug substance having controlled particle size is more likely to be properly mixed with additives. Thus, with such a drug substance, highly uniform pharmaceutical compositions and pharmaceutical formulations, which also have the stability of drug substance therein, can be obtained (Patent Literature 8). Encapsulation of highly uniform filler powder into a capsule shell enables the efficient dispersion of the filler powder in the gastrointestinal tract after dissolution of the capsule shell, and promotes the absorption of the active ingredient. In addition, it is required in the quality control of formulations that the uniformity of mixed filler powders is maintained above a certain level.

### CITATION LIST

### PATENT LITERATURE

[Patent Literature 1] International Publication No. WO 2012/006475
[Patent Literature 2] International Publication No. WO 2011/136269
[Patent Literature 3] International Publication No. WO 2013/062065
[Patent Literature 4] International Publication No. WO 2014/003153
[Patent Literature 5] International Publication No. WO 2018/034883
[Patent Literature 6] International Publication No. WO 2014/142273
[Patent Literature 7] International Publication No. WO 2016/039458
[Patent Literature 8] International Publication No. WO 2016/026822

### NON PATENT LITERATURE

[Non Patent Literature 1] Hruska, KA et al., Kidney Int., 2008, 74(2), 148-157.
[Non Patent Literature 2] "Chapter 1: Definition and classification of CKD" Kidney Int. Suppl., 2013, 3(1), 19-62.
[Non Patent Literature 3] Levey, AS et al., Kidney Int., 2005, 67(6), 2089-2100.
[Non Patent Literature 4] Ritter, CS et al., Clin. J.Am.Soc.Nephrol.2016, 11(6), 1088-1100.
[Non Patent Literature 5] Miyamoto, K et al., J. Pharm. Sci., 2011, 100(9), 3719-3730.
[Non Patent Literature 6] Sabbagh, Y et al., J. Am. Soc. Nephrol., 2009, 20(11), 2348-2358.
[Non Patent Literature 7] Forster IC et al., Mol Aspects Med., 2013, 34(2-3), 386-395.
[Non Patent Literature 8] Lederer E et al., Eur. J. Physiol., 2019, 471(1), 137-148.
[Non Patent Literature 9] Japan Institute for Promoting Invention and Innovation, Journal of technical disclosure, Technique No. 2017-501666

### SUMMARY OF INVENTION

### TECHNICAL PROBLEM

In the pharmaceutical composition and pharmaceutical formulation containing the compound represented by formula 1 or a salt thereof or a solvate of these as an active ingredient, no pharmaceutical compositions and pharmaceutical formulations capable of exerting a sufficient medicinal effect have been known due to the low solubility of the active ingredient. Specifically, in the pharmaceutical compositions and pharmaceutical formulations containing a p-toluenesulfonate of 7-[[2,3-difluoro-4-[2-[2-methoxyethyl(methyl)amino]ethoxy]phenyl]methyl]-10-hydroxy-6-methyl-8-oxo-N-[4-(trifluoromethyl)-2-[6-(trifluoromethyl)pyrimidin-4-yl]phenyl]-6,7-diazaspiro[4,5]deca-9-ene-9-carboxamide (compound I) as an active ingredient, a dissolution delay of the active ingredient has been observed in the pharmaceutical compositions and pharmaceutical formulations in which unpulverized products of the drug substance are used. When a surfactant is added to the pharmaceutical composition to improve the solubility of the active ingredient in water, it has been observed that stirring becomes poor and the uniformity of the obtained powder mixture (bulk powder), which is the pharmaceutical composition of the pharmaceutical formulation raw material containing compound I, becomes insufficient due to the high viscosity of the bulk powder during the stirring of the bulk powder to which the surfactant is added.

### SOLUTION TO PROBLEM

The present inventors have extensively conducted studies to solve these problems. Then, the present inventors have found that the dissolution delay of the active ingredient is reduced by using a specific additive and a drug substance having a specific particle size. In addition, the present inventors have found that the use of a specific lubricant as an additive yields the bulk powder mixed well without causing poor stirring due to the high viscosity of the bulk powder. Furthermore, the present inventors have found that the additional use of one or more additives selected from specific excipients and disintegrants enables stable production of the bulk powder having uniformity of or above a certain level without causing poor stirring due to the high viscosity of the bulk powder. In addition, the present inventors have found, based on the bulk powder thus obtained, a pharmaceutical composition and a pharmaceutical formulation from which the active ingredient is efficiently dissolved. Specifically, it has been found that, from a capsule containing a bulk powder encapsulating compound I, which is an active ingredient, as a filler, the active ingredient is efficiently dissolved. Based on these findings, the present inventors have completed the present invention.

Specifically, the disclosure of the following invention is encompassed herein.
[1-1] A pharmaceutical composition containing:
   a compound represented by formula 1: 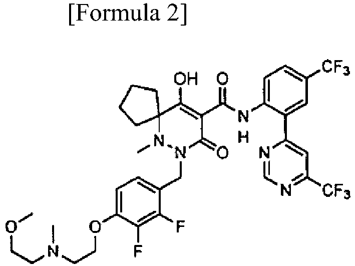
   or a salt thereof or a solvate of these; and a lubricant.
[1-2] The pharmaceutical composition according to [1-1], further containing an excipient and a disintegrant.
[1-3] The pharmaceutical composition according to [1-1] or [1-2], wherein a content of the lubricant is 0.5 wt% or more based on a total of the pharmaceutical composition.
[1-4] The pharmaceutical composition according to any of [1-1] to [1-3], wherein a content of the lubricant is in a range of 0.5 wt% or more and 15 wt% or less based on the total of the pharmaceutical composition.
[1-5] The pharmaceutical composition according to any of [1-1] to [1-4], wherein a content of the lubricant is 6.0 wt% or more based on the total of the pharmaceutical composition.
[1-6] The pharmaceutical composition according to any of [1-1] to [1-5], wherein a content of the lubricant is 7.0 wt% or more based on the total of the pharmaceutical composition.
[1-7] The pharmaceutical composition according to any of [1-1] to [1-6], wherein a content of the lubricant is in a range of 5.3 wt% or more and 15 wt% or less based on the total of the pharmaceutical composition.
[1-8] The pharmaceutical composition according to any of [1-1] to [1-7], wherein a content of the lubricant is in a range of 6.0 wt% or more and 13 wt% or less based on the total of the pharmaceutical composition.
[1-9] The pharmaceutical composition according to any of [1-1] to [1-8], wherein a content of the lubricant is in a range of 7.0 wt% or more and 11 wt% or less based on the total of the pharmaceutical composition.
[1-10] The pharmaceutical composition according to any of [1-2] to [1-9], wherein a content of the excipient is 21 wt% or more based on the total of the pharmaceutical composition.
[1-11] The pharmaceutical composition according to any of [1-2] to [1-10], wherein a content of the excipient is in a range of 21 wt% or more and 72 wt% or less based on the total of the pharmaceutical composition.
[1-12] The pharmaceutical composition according to any of [1-2] to [1-11], wherein a content of the excipient is in a range of 27 wt% or more and 66 wt% or less based on the total of the pharmaceutical composition.
[1-13] The pharmaceutical composition according to any of [1-2] to [1-12], wherein a content of the disintegrant is 10 wt% or more based on the total of the pharmaceutical composition.
[1-14] The pharmaceutical composition according to any of [1-2] to [1-13], wherein a content of the disintegrant is in a range of 10 wt% or more and 30 wt% or less based on the total of the pharmaceutical composition.
[1-15] The pharmaceutical composition according to any of [1-2] to [1-14], wherein a content of the disintegrant is in a range of 18 wt% or more and 22 wt% or less based on the total of the pharmaceutical composition.
[1-16] The pharmaceutical composition according to any of [1-1] to [1-15], wherein, as the lubricant, at least one lubricant selected from the group consisting of sodium stearyl fumarate, zinc stearate, aluminum stearate, calcium stearate, magnesium stearate, talc, and a sucrose fatty acid ester is contained.
[1-17] The pharmaceutical composition according to any of [1-1] to [1-16], wherein sodium stearyl fumarate, magnesium stearate, calcium stearate, or talc is contained as the lubricant.
[1-18] The pharmaceutical composition according to any of [1-1] to [1-17], wherein sodium stearyl fumarate is contained as the lubricant.
[1-19] The pharmaceutical composition according to any of [1-2] to [1-18], wherein, as the excipient, at least one excipient selected from the group consisting of mannitol, lactose hydrate, fructose, glucose, sorbitol, corn starch, potato starch, wheat starch, and rice starch is contained.
[1-20] The pharmaceutical composition according to any of [1-2] to [1-19], wherein mannitol or lactose hydrate is contained as the excipient.
[1-21] The pharmaceutical composition according to any of [1-2] to [1-20], wherein mannitol is contained as the excipient.
[1-22] The pharmaceutical composition according to any of [1-2] to [1-21], wherein, as the disintegrant, at least one disintegrant selected from the group consisting of croscarmellose sodium, carmellose sodium, hydroxypropyl cellulose, carmellose, carmellose calcium, methylcellulose, crystalline cellulose, sodium lauryl sulfate, povidone, and polysorbate is contained.
[1-23] The pharmaceutical composition according to any of [1-2] to [1-22], wherein croscarmellose sodium, carmellose sodium, carmellose calcium, or hydroxypropyl cellulose is contained as the disintegrant.
[1-24] The pharmaceutical composition according to any of [1-2] to [1-23], wherein croscarmellose sodium or carmellose calcium is contained as the disintegrant.
[1-25] The pharmaceutical composition according to any of [1-1] to [1-24], wherein a content of the compound represented by formula 1 or a salt thereof or a solvate of these is in a range of 1 wt% or more and 65 wt% or less based on the total of the pharmaceutical composition.
[1-26] The pharmaceutical composition according to any of [1-1] to [1-25], wherein a content of the compound represented by formula 1 or a salt thereof or a solvate of these is in a range of 8 wt% or more and 45 wt% or less based on the total of the pharmaceutical composition.
[1-27] The pharmaceutical composition according to any of [1-1] to [1-25], wherein the compound represented by formula 1 or a salt thereof or a solvate of these is a compound represented by formula 1 or a salt thereof.
[1-28] The pharmaceutical composition according to any of [1-1] to [1-25], wherein the compound represented by formula 1 or a salt thereof or a solvate of these is a salt of the compound represented by formula 1.
[1-29] The pharmaceutical composition according to any of [1-1] to [1-25], wherein the compound represented by formula 1 or a salt thereof or a solvate of these is a compound represented by formula 1.
[1-30] The pharmaceutical composition according to any of [1-1] to [1-28], wherein the compound represented by formula 1 or a salt thereof or a solvate of these is a p-toluenesulfonate of the compound represented by formula 1.
[1-31] The pharmaceutical composition according to [1-30], wherein the p-toluenesulfonate of the compound represented by formula 1 is a crystal.
[1-32] The pharmaceutical composition according to [1-30] or [1-31], wherein the p-toluenesulfonate of the compound represented by formula 1 is a type 1 crystal.
[1-33] The pharmaceutical composition according to [1-31] or [1-32], wherein an X-ray powder diffraction pattern of the crystal has a peak at at least one diffraction angle (2θ) selected from the group consisting of 4.9°, 9.4°, 9.9°, 15.2°, 15.8°, 18.9° and 22.6° (± 0.2°).
[1-34] The pharmaceutical composition according to any of [1-31] to [1-33], wherein an X-ray powder diffraction pattern of the crystal has a peak at at least two diffraction angles (2θ) selected from the group consisting of 4.9°, 9.4°, 9.9°, 15.2°, 15.8°, 18.9° and 22.6° (± 0.2°).
[1-35] The pharmaceutical composition according to [1-34], wherein an X-ray powder diffraction pattern of the crystal has a peak at diffraction angles (2θ) of
   4.9° and 9.4° (± 0.2°),
   4.9° and 9.9° (± 0.2°),
   4.9° and 15.2° (± 0.2°),
   4.9° and 15.8° (± 0.2°),
   4.9° and 18.9° (± 0.2°),
   4.9° and 22.6° (± 0.2°),
   9.4° and 9.9° (± 0.2°),
   9.4° and 15.2° (± 0.2°),
   9.4° and 15.8° (± 0.2°),
   9.4° and 18.9° (± 0.2°),
   9.4° and 22.6° (± 0.2°),
   9.9° and 15.2° (± 0.2°),
   9.9° and 15.8° (± 0.2°),
   9.9° and 18.9° (± 0.2°),
   9.9° and 22.6° (± 0.2°),
   15.2° and 15.8° (± 0.2°),
   15.2° and 18.9° (± 0.2°),
   15.2° and 22.6° (± 0.2°),
   15.8° and 18.9° (± 0.2°),
   15.8° and 22.6° (± 0.2°), or
   18.9° and 22.6° (± 0.2°).
[1-36] The pharmaceutical composition according to any of [1-31] to [1-35], wherein an X-ray powder diffraction pattern of the crystal has a peak at at least three diffraction angles (2θ) selected from the group consisting of 4.9°, 9.4°, 9.9°, 15.2°, 15.8°, 18.9° and 22.6° (± 0.2°).
[1-37] The pharmaceutical composition according to [1-36], wherein an X-ray powder diffraction pattern of the crystal has a peak at diffraction angles (2θ) of
   4.9°, 9.4°, and 9.9° (± 0.2°),
   4.9°, 9.4°, and 15.2° (± 0.2°),
   4.9°, 9.4°, and 15.8° (± 0.2°),
   4.9°, 9.4°, and 18.9° (± 0.2°),
   4.9°, 9.4°, and 22.6° (± 0.2°),
   4.9°, 9.9°, and 15.2° (± 0.2°),
   4.9°, 9.9°, and 15.8° (± 0.2°),
   4.9°, 9.9°, and 18.9° (± 0.2°),
   4.9°, 9.9°, and 22.6° (± 0.2°),
   4.9°, 15.2°, and 15.8° (± 0.2°),
   4.9°, 15.2°, and 18.9° (± 0.2°),
   4.9°, 15.2°, and 22.6° (± 0.2°),
   4.9°, 15.8°, and 18.9° (± 0.2°),
   4.9°, 15.8°, and 22.6° (± 0.2°),
   4.9°, 18.9°, and 22.6° (± 0.2°),
   9.4°, 9.9°, and 15.2° (± 0.2°),
   9.4°, 9.9°, and 15.8° (± 0.2°),
   9.4°, 9.9°, and 18.9° (± 0.2°),
   9.4°, 9.9°, and 22.6° (± 0.2°),
   9.4°, 15.2°, and 15.8° (± 0.2°),
   9.4°, 15.2°, and 18.9° (± 0.2°),
   9.4°, 15.2°, and 22.6° (± 0.2°),
   9.4°, 15.8°, and 18.9° (± 0.2°),
   9.4°, 15.8°, and 22.6° (± 0.2°),
   9.4°, 18.9°, and 22.6° (± 0.2°),
   9.9°, 15.2°, and 15.8° (± 0.2°),
   9.9°, 15.2°, and 18.9° (± 0.2°),
   9.9°, 15.2°, and 22.6° (± 0.2°),
   9.9°, 15.8°, and 18.9° (± 0.2°),
   9.9°, 15.8°, and 22.6° (± 0.2°),
   9.9°, 18.9°, and 22.6° (± 0.2°),
   15.2°, 15.8°, and 18.9° (± 0.2°),
   15.2°, 15.8°, and 22.6° (± 0.2°),
   15.2°, 18.9°, and 22.6° (± 0.2°), or
   15.8°, 18.9°, and 22.6°.
[1-38] The pharmaceutical composition according to any of [1-31] to [1-37], wherein an X-ray powder diffraction pattern of the crystal has a peak at at least four diffraction angles (2θ) selected from the group consisting of 4.9°, 9.4°, 9.9°, 15.2°, 15.8°, 18.9° and 22.6° (± 0.2°).
[1-39] The pharmaceutical composition according to [1-38], wherein an X-ray powder diffraction pattern of the crystal has a peak at diffraction angles (2θ) of
   4.9°, 9.4°, 9.9°, and 15.2° (± 0.2°),
   4.9°, 9.4°, 9.9°, and 15.8° (± 0.2°),
   4.9°, 9.4°, 9.9°, and 18.9° (± 0.2°),
   4.9°, 9.4°, 9.9°, and 22.6° (± 0.2°),
   4.9°, 9.4°, 15.2°, and 15.8° (± 0.2°),
   4.9°, 9.4°, 15.2°, and 18.9° (± 0.2°),
   4.9°, 9.4°, 15.2°, and 22.6° (± 0.2°),
   4.9°, 9.4°, 15.8°, and 18.9° (± 0.2°),
   4.9°, 9.4°, 15.8°, and 22.6° (± 0.2°),
   4.9°, 9.4°, 18.9°, and 22.6° (± 0.2°),
   4.9°, 9.9°, 15.2°, and 15.8° (± 0.2°),
   4.9°, 9.9°, 15.2°, and 18.9° (± 0.2°),
   4.9°, 9.9°, 15.2°, and 22.6° (± 0.2°),
   4.9°, 9.9°, 15.8°, and 18.9° (± 0.2°),
   4.9°, 9.9°, 15.8°, and 22.6° (± 0.2°),
   4.9°, 9.9°, 18.9°, and 22.6° (± 0.2°),
   4.9°, 15.2°, 15.8°, and 18.9° (± 0.2°),
   4.9°, 15.2°, 15.8°, and 22.6° (± 0.2°),
   4.9°, 15.2°, 18.9°, and 22.6° (± 0.2°),
   4.9°, 15.8°, 18.9°, and 22.6° (± 0.2°),
   9.4°, 9.9°, 15.2°, and 15.8° (± 0.2°),
   9.4°, 9.9°, 15.2°, and 18.9° (± 0.2°),
   9.4°, 9.9°, 15.2°, and 22.6° (± 0.2°),
   9.4°, 9.9°, 15.8°, and 18.9° (± 0.2°),
   9.4°, 9.9°, 15.8°, and 22.6° (± 0.2°),
   9.4°, 9.9°, 18.9°, and 22.6° (± 0.2°),
   9.4°, 15.2°, 15.8°, and 18.9° (± 0.2°),
   9.4°, 15.2°, 15.8°, and 22.6° (± 0.2°),
   9.4°, 15.2°, 18.9°, and 22.6° (± 0.2°),
   9.4°, 15.8°, 18.9°, and 22.6° (± 0.2°),
   9.9°, 15.2°, 15.8°, and 18.9° (± 0.2°),
   9.9°, 15.2°, 15.8°, and 22.6° (± 0.2°),
   9.9°, 15.2°, 18.9°, and 22.6° (± 0.2°),
   9.9°, 15.8°, 18.9°, and 22.6° (± 0.2°), or
   15.2°, 15.8°, 18.9° and 22.6° (± 0.2°).
[1-40] The pharmaceutical composition according to any of [1-31] to [1-39], wherein an X-ray powder diffraction pattern of the crystal has a peak at at least five diffraction angles (2θ) selected from the group consisting of 4.9°, 9.4°, 9.9°, 15.2°, 15.8°, 18.9° and 22.6° (± 0.2°).
[1-41] The pharmaceutical composition according to [1-40], wherein an X-ray powder diffraction pattern of the crystal has a peak at diffraction angles (2θ) of
   4.9°, 9.4°, 9.9°, 15.2°, and 15.8° (± 0.2°),
   4.9°, 9.4°, 9.9°, 15.2°, and 18.9° (± 0.2°),
   4.9°, 9.4°, 9.9°, 15.2°, and 22.6° (± 0.2°),
   4.9°, 9.4°, 9.9°, 15.8°, and 18.9° (± 0.2°),
   4.9°, 9.4°, 9.9°, 15.8°, and 22.6° (± 0.2°),
   4.9°, 9.4°, 9.9°, 18.9°, and 22.6° (± 0.2°),
   4.9°, 9.4°, 15.2°, 15.8°, and 18.9° (± 0.2°),
   4.9°, 9.4°, 15.2°, 15.8°, and 22.6° (± 0.2°),
   4.9°, 9.4°, 15.2°, 18.9°, and 22.6° (± 0.2°),
   4.9°, 9.4°, 15.8°, 18.9°, and 22.6° (± 0.2°),
   4.9°, 9.9°, 15.2°, 15.8°, and 18.9° (± 0.2°),
   4.9°, 9.9°, 15.2°, 15.8°, and 22.6° (± 0.2°),
   4.9°, 9.9°, 15.2°, 18.9°, and 22.6° (± 0.2°),
   4.9°, 9.9°, 15.8°, 18.9°, and 22.6° (± 0.2°),
   4.9°, 15.2°, 15.8°, 18.9°, and 22.6° (± 0.2°),
   9.4°, 9.9°, 15.2°, 15.8°, and 18.9° (± 0.2°),
   9.4°, 9.9°, 15.2°, 15.8°, and 22.6° (± 0.2°),
   9.4°, 9.9°, 15.2°, 18.9°, and 22.6° (± 0.2°),
   9.4°, 9.9°, 15.8°, 18.9°, and 22.6° (± 0.2°),
   9.4°, 15.2°, 15.8°, 18.9°, and 22.6° (± 0.2°), or
   9.9°, 15.2°, 15.8°, 18.9° and 22.6° (± 0.2°).
[1-42] The pharmaceutical composition according to any of [1-31] to [1-41], wherein an X-ray powder diffraction pattern of the crystal has a peak at at least six diffraction angles (2θ) selected from the group consisting of 4.9°, 9.4°, 9.9°, 15.2°, 15.8°, 18.9° and 22.6° (± 0.2°).
[1-43] The pharmaceutical composition according to [1-42], wherein an X-ray powder diffraction pattern of the crystal has a peak at diffraction angles (2θ) of
   4.9°, 9.4°, 9.9°, 15.2°, 15.8°, and 18.9° (± 0.2°),
   4.9°, 9.4°, 9.9°, 15.2°, 15.8°, and 22.6° (± 0.2°),
   4.9°, 9.4°, 9.9°, 15.2°, 18.9°, and 22.6° (± 0.2°),
   4.9°, 9.4°, 9.9°, 15.8°, 18.9°, and 22.6° (± 0.2°),
   4.9°, 9.4°, 15.2°, 15.8°, 18.9°, and 22.6° (± 0.2°),
   4.9°, 9.9°, 15.2°, 15.8°, 18.9°, and 22.6° (± 0.2°), or
   9.4°, 9.9°, 15.2°, 15.8°, 18.9° and 22.6° (± 0.2°).
[1-44] The pharmaceutical composition according to any of [1-31] to [1-43], wherein an X-ray powder diffraction pattern of the crystal has a peak at diffraction angles (2θ) of 4.9°, 9.4°, 9.9°, 15.2°, 15.8°, 18.9° and 22.6° (± 0.2°).
[1-45] The pharmaceutical composition according to any of [1-31] to [1-44], wherein a volume-based particle size of the crystal of the p-toluenesulfonate of the compound represented by formula 1 has d₁₀ of less than 5.76 µm, d₅₀ of less than 8.81 µm, or d₉₀ of less than 13.08 µm.
[1-46] The pharmaceutical composition according to any of [1-31] to [1-45], wherein a volume-based particle size of the crystal of the p-toluenesulfonate of the compound represented by formula 1 has d₁₀ of 5.00 µm or less, d₅₀ of 8.00 µm or less, or d₉₀ of 12.00 µm or less.
[1-47] The pharmaceutical composition according to any of [1-31] to [1-46], wherein a volume-based particle size of the crystal of the p-toluenesulfonate of the compound represented by formula 1 has d₁₀ of 4.00 µm or less, d₅₀ of 6.00 µm or less, or d₉₀ of 11.00 µm or less.
[1-48] The pharmaceutical composition according to any of [1-31] to [1-47], wherein a volume-based particle size of the crystal of the p-toluenesulfonate of the compound represented by formula 1 has d₁₀ of 3.00 µm or less, d₅₀ of 5.00 µm or less, or d₉₀ of 8.00 µm or less.
[1-49] The pharmaceutical composition according to any of [1-31] to [1-48], wherein a volume-based particle size of the crystal of the p-toluenesulfonate of the compound represented by formula 1 has d₁₀ of 1.50 µm or more and less than 5.76 µm, d₅₀ of 2.60 µm or more and less than 8.81 µm, or d₉₀ of 4.80 µm or more and less than 13.08 µm.
[1-50] The pharmaceutical composition according to any of [1-31] to [1-49], wherein a volume-based particle size of the crystal of the p-toluenesulfonate of the compound represented by formula 1 has d₁₀ of 1.90 µm or more and less than 5.76 µm, d₅₀ of 3.40 µm or more and less than 8.81 µm, or d₉₀ of 5.80 µm or more and less than 13.08 µm.
[1-51] The pharmaceutical composition according to any of [1-31] to [1-50], wherein a volume-based particle size of the crystal of the p-toluenesulfonate of the compound represented by formula 1 has d₁₀ of 2.00 µm or more and less than 5.76 µm, d₅₀ of 3.50 µm or more and less than 8.81 µm, or d₉₀ of 5.90 µm or more and less than 13.08 µm.
[1-52] The pharmaceutical composition according to any of [1-31] to [1-51], wherein a volume-based particle size of the crystal of the p-toluenesulfonate of the compound represented by formula 1 has d₁₀ of less than 5.76 µm, d₅₀ of less than 8.81 µm, and d₉₀ of less than 13.08 µm.
[1-53] The pharmaceutical composition according to any of [1-31] to [1-52], wherein a volume-based particle size of the crystal of the p-toluenesulfonate of the compound represented by formula 1 has d₁₀ of 5.00 µm or less, d₅₀ of 8.00 µm or less, and d₉₀ of 12.00 µm or less.
[1-54] The pharmaceutical composition according to any of [1-31] to [1-53], wherein a volume-based particle size of the crystal of the p-toluenesulfonate of the compound represented by formula 1 has d₁₀ of 4.00 µm or less, d₅₀ of 6.00 µm or less, and d₉₀ of 11.00 µm or less.
[1-55] The pharmaceutical composition according to any of [1-31] to [1-54], wherein a volume-based particle size of the crystal of the p-toluenesulfonate of the compound represented by formula 1 has d₁₀ of 3.00 µm or less, d₅₀ of 5.00 µm or less, and d₉₀ of 8.00 µm or less.
[1-56] The pharmaceutical composition according to any of [1-31] to [1-55], wherein a volume-based particle size of the crystal of the p-toluenesulfonate of the compound represented by formula 1 has d₁₀ of 1.00 µm or more and less than 5.76 µm, d₅₀ of 2.30 µm or more and less than 8.81 µm, and d₉₀ of 4.30 µm or more and less than 13.08 µm.
[1-57] The pharmaceutical composition according to any of [1-31] to [1-56], wherein a volume-based particle size of the crystal of the p-toluenesulfonate of the compound represented by formula 1 has d₁₀ of 1.20 µm or more and less than 5.76 µm, d₅₀ of 2.40 µm or more and less than 8.81 µm, and d₉₀ of 4.50 µm or more and less than 13.08 µm.
[1-58] The pharmaceutical composition according to any of [1-31] to [1-57], wherein a volume-based particle size of the crystal of the p-toluenesulfonate of the compound represented by formula 1 has d₁₀ of 1.30 µm or more and less than 5.76 µm, d₅₀ of 2.50 µm or more and less than 8.81 µm, and d₉₀ of 4.70 µm or more and less than 13.08 µm.
[1-59] The pharmaceutical composition according to any of [1-1] to [1-58], wherein the pharmaceutical composition is a powder containing a p-toluenesulfonate of the compound represented by formula 1, which is an active ingredient, as a type 1 crystal, and further containing mannitol, croscarmellose sodium, and sodium stearyl fumarate.
[1-60] A pharmaceutical formulation containing the pharmaceutical composition according to any of [1-1] to [1-59].
[1-61] The pharmaceutical formulation according to [1-60], wherein a dosage form of the pharmaceutical formulation is selected from the group consisting of a powdered formulation, a powder, a granule, a tablet, and a capsule.
[1-62] The pharmaceutical formulation according to [1-60] or [1-61], wherein a dosage form of the pharmaceutical formulation is a powdered formulation or a capsule.
[1-63] The pharmaceutical formulation according to any of [1-60] to [1-62], wherein the pharmaceutical formulation is in a form of a capsule containing a p-toluenesulfonate of the compound represented by formula 1, which is an active ingredient, as a type 1 crystal, and further containing mannitol, croscarmellose sodium, and sodium stearyl fumarate.
[1-64] The pharmaceutical composition according to [1-59], wherein the powder is used as a filler of a capsule.
[2-1] A pharmaceutical formulation containing a pharmaceutical composition containing a p-toluenesulfonate of a compound represented by formula 1: 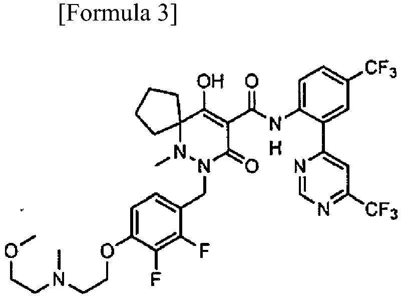 , and a lubricant.
[2-2] The pharmaceutical formulation according to [2-1], wherein the pharmaceutical composition further contains an excipient and a disintegrant.
[2-3] The pharmaceutical formulation according to [2-1] or [2-2], wherein a dosage form of the pharmaceutical formulation is selected from the group consisting of a powdered formulation, a powder, a granule, a tablet, and a capsule.
[2-4] The pharmaceutical formulation according to any of [2-1] to [2-3], wherein a dosage form of the pharmaceutical formulation is a powdered formulation or a capsule.
[2-5] The pharmaceutical formulation according to any of [2-1] to [2-4], wherein a dosage form of the pharmaceutical formulation is a capsule.
[2-6] The pharmaceutical formulation according to any of [2-1] to [2-5], wherein the capsule contains a powder of the pharmaceutical composition.
[2-7] The pharmaceutical formulation according to any of [2-1] to [2-6], wherein a content of the lubricant is 0.5 wt% or more based on a total of the pharmaceutical composition.
[2-8] The pharmaceutical formulation according to any of [2-1] to [2-7], wherein a content of the lubricant is in a range of 0.5 wt% or more and 15 wt% or less based on the total of the pharmaceutical composition.
[2-9] The pharmaceutical formulation according to any of [2-1] to [2-8], wherein a content of the lubricant is 6 wt% or more based on the total of the pharmaceutical composition.
[2-10] The pharmaceutical formulation according to any of [2-1] to [2-9], wherein a content of the lubricant is 7 wt% or more based on the total of the pharmaceutical composition.
[2-11] The pharmaceutical formulation according to any of [2-1] to [2-10], wherein a content of the lubricant is in a range of 5.3 wt% or more and 15 wt% or less based on the total of the pharmaceutical composition.
[2-12] The pharmaceutical formulation according to any of [2-1] to [2-11], wherein a content of the lubricant is in a range of 6 wt% or more and 11 wt% or less based on the total of the pharmaceutical composition.
[2-13] The pharmaceutical formulation according to any of [2-2] to [2-12], wherein a content of the excipient is 21 wt% or more based on the total of the pharmaceutical composition.
[2-14] The pharmaceutical formulation according to any of [2-2] to [2-13], wherein a content of the excipient is in a range of 21 wt% or more and 72 wt% or less based on the total of the pharmaceutical composition.
[2-15] The pharmaceutical formulation according to any of [2-2] to [2-14], wherein a content of the excipient is in a range of 27 wt% or more and 66 wt% or less based on the total of the pharmaceutical composition.
[2-16] The pharmaceutical formulation according to any of [2-2] to [2-15], wherein a content of the disintegrant is 10 wt% or more based on the total of the pharmaceutical composition.
[2-17] The pharmaceutical formulation according to any of [2-2] to [2-16], wherein a content of the disintegrant is in a range of 10 wt% or more and 30 wt% or less based on the total of the pharmaceutical composition.
[2-18] The pharmaceutical formulation according to any of [2-2] to [2-17], wherein a content of the disintegrant is in a range of 18 wt% or more and 22 wt% or less based on the total of the pharmaceutical composition.
[2-19] The pharmaceutical formulation according to any of [2-1] to [2-18], wherein, as the lubricant, at least one lubricant selected from the group consisting of sodium stearyl fumarate, zinc stearate, aluminum stearate, calcium stearate, magnesium stearate, talc, and a sucrose fatty acid ester is contained.
[2-20] The pharmaceutical formulation according to any of [2-1] to [2-19], wherein sodium stearyl fumarate, magnesium stearate, calcium stearate, or talc is contained as the lubricant.
[2-21] The pharmaceutical formulation according to any of [2-1] to [2-20], wherein sodium stearyl fumarate is contained as the lubricant.
[2-22] The pharmaceutical formulation according to any of [2-2] to [2-21], wherein, as the excipient, at least one excipient selected from the group consisting of mannitol, lactose hydrate, fructose, glucose, sorbitol, corn starch, potato starch, wheat starch, and rice starch is contained.
[2-23] The pharmaceutical formulation according to any of [2-2] to [2-22], wherein mannitol or lactose hydrate is contained as the excipient.
[2-24] The pharmaceutical formulation according to any of [2-2] to [2-23], wherein mannitol is contained as the excipient.
[2-25] The pharmaceutical formulation according to any of [2-2] to [2-24], wherein, as the disintegrant, at least one disintegrant selected from the group consisting of croscarmellose sodium, carmellose sodium, hydroxypropyl cellulose, carmellose, carmellose calcium, methylcellulose, crystalline cellulose, sodium lauryl sulfate, povidone, and polysorbate is contained.
[2-26] The pharmaceutical formulation according to any of [2-2] to [2-25], wherein croscarmellose sodium, carmellose sodium, carmellose calcium, or hydroxypropyl cellulose is contained as the disintegrant.
[2-27] The pharmaceutical formulation according to any of [2-2] to [2-26], wherein croscarmellose sodium or carmellose calcium is contained as the disintegrant.
[2-28] The pharmaceutical formulation according to any of [2-1] to [2-27], wherein a content of the p-toluenesulfonate is in a range of 1 wt% or more and 65 wt% or less based on the total of the pharmaceutical composition.
[2-29] The pharmaceutical formulation according to any of [2-1] to [2-28], wherein a content of the p-toluenesulfonate is in a range of 8 wt% or more and 45 wt% or less based on the total of the pharmaceutical composition.
[2-30] The pharmaceutical formulation according to any of [2-1] to [2-28], wherein the compound represented by formula 1 or a salt thereof or a solvate of these is a compound represented by formula 1 or a salt thereof.
[2-31] The pharmaceutical formulation according to any of [2-1] to [2-28], wherein the compound represented by formula 1 or a salt thereof or a solvate of these is a salt of the compound represented by formula 1.
[2-32] The pharmaceutical formulation according to any of [2-1] to [2-27], wherein the compound represented by formula 1 or a salt thereof or a solvate of these is a compound represented by formula 1.
[2-33] The pharmaceutical formulation according to any of [2-1] to [2-28], wherein the compound represented by formula 1 or a salt thereof or a solvate of these is a p-toluenesulfonate of the compound represented by formula 1.
[2-34] The pharmaceutical formulation according to any of [2-1] to [2-33], wherein the p-toluenesulfonate of the compound is a crystal.
[2-35] The pharmaceutical formulation according to any of [2-1] to [2-34], wherein the p-toluenesulfonate of the compound represented by formula 1 is a type 1 crystal.
[2-36] The pharmaceutical formulation according to [2-34] or [2-35], wherein an X-ray powder diffraction pattern of the crystal has a peak at at least one diffraction angle (2θ) selected from the group consisting of 4.9°, 9.4°, 9.9°, 15.2°, 15.8°, 18.9° and 22.6° (± 0.2°).
[2-37] The pharmaceutical formulation according to any of [2-34] to [2-36], wherein an X-ray powder diffraction pattern of the crystal has a peak at at least two diffraction angles (2θ) selected from the group consisting of 4.9°, 9.4°, 9.9°, 15.2°, 15.8°, 18.9° and 22.6° (± 0.2°).
[2-38] The pharmaceutical formulation according to [2-37], wherein an X-ray powder diffraction pattern of the crystal has a peak at diffraction angles (2θ) of
   4.9° and 9.4° (± 0.2°),
   4.9° and 9.9° (± 0.2°),
   4.9° and 15.2° (± 0.2°),
   4.9° and 15.8° (± 0.2°),
   4.9° and 18.9° (± 0.2°),
   4.9° and 22.6° (± 0.2°),
   9.4° and 9.9° (± 0.2°),
   9.4° and 15.2° (± 0.2°),
   9.4° and 15.8° (± 0.2°),
   9.4° and 18.9° (± 0.2°),
   9.4° and 22.6° (± 0.2°),
   9.9° and 15.2° (± 0.2°),
   9.9° and 15.8° (± 0.2°),
   9.9° and 18.9° (± 0.2°),
   9.9° and 22.6° (± 0.2°),
   15.2° and 15.8° (± 0.2°),
   15.2° and 18.9° (± 0.2°),
   15.2° and 22.6° (± 0.2°),
   15.8° and 18.9° (± 0.2°),
   15.8° and 22.6° (± 0.2°), or
   18.9° and 22.6° (± 0.2°).
[2-39] The pharmaceutical formulation according to any of [2-33] to [2-38], wherein an X-ray powder diffraction pattern of the crystal has a peak at at least three diffraction angles (2θ) selected from the group consisting of 4.9°, 9.4°, 9.9°, 15.2°, 15.8°, 18.9° and 22.6° (± 0.2°).
[2-40] The pharmaceutical formulation according to [2-39], wherein an X-ray powder diffraction pattern of the crystal has a peak at diffraction angles (2θ) of
   4.9°, 9.4°, and 9.9° (± 0.2°),
   4.9°, 9.4°, and 15.2° (± 0.2°),
   4.9°, 9.4°, and 15.8° (± 0.2°),
   4.9°, 9.4°, and 18.9° (± 0.2°),
   4.9°, 9.4°, and 22.6° (± 0.2°),
   4.9°, 9.9°, and 15.2° (± 0.2°),
   4.9°, 9.9°, and 15.8° (± 0.2°),
   4.9°, 9.9°, and 18.9° (± 0.2°),
   4.9°, 9.9°, and 22.6° (± 0.2°),
   4.9°, 15.2°, and 15.8° (± 0.2°),
   4.9°, 15.2°, and 18.9° (± 0.2°),
   4.9°, 15.2°, and 22.6° (± 0.2°),
   4.9°, 15.8°, and 18.9° (± 0.2°),
   4.9°, 15.8°, and 22.6° (± 0.2°),
   4.9°, 18.9°, and 22.6° (± 0.2°),
   9.4°, 9.9°, and 15.2° (± 0.2°),
   9.4°, 9.9°, and 15.8° (± 0.2°),
   9.4°, 9.9°, and 18.9° (± 0.2°),
   9.4°, 9.9°, and 22.6° (± 0.2°),
   9.4°, 15.2°, and 15.8° (± 0.2°),
   9.4°, 15.2°, and 18.9° (± 0.2°),
   9.4°, 15.2°, and 22.6° (± 0.2°),
   9.4°, 15.8°, and 18.9° (± 0.2°),
   9.4°, 15.8°, and 22.6° (± 0.2°),
   9.4°, 18.9°, and 22.6° (± 0.2°),
   9.9°, 15.2°, and 15.8° (± 0.2°),
   9.9°, 15.2°, and 18.9° (± 0.2°),
   9.9°, 15.2°, and 22.6° (± 0.2°),
   9.9°, 15.8°, and 18.9° (± 0.2°),
   9.9°, 15.8°, and 22.6° (± 0.2°),
   9.9°, 18.9°, and 22.6° (± 0.2°),
   15.2°, 15.8°, and 18.9° (± 0.2°),
   15.2°, 15.8°, and 22.6° (± 0.2°),
   15.2°, 18.9°, and 22.6° (± 0.2°), or
   15.8°, 18.9° and 22.6° (± 0.2°).
[2-41] The pharmaceutical formulation according to any of [2-33] to [2-40], wherein an X-ray powder diffraction pattern of the crystal has a peak at at least four diffraction angles (2θ) selected from the group consisting of 4.9°, 9.4°, 9.9°, 15.2°, 15.8°, 18.9° and 22.6° (± 0.2°).
[2-42] The pharmaceutical formulation according to [2-41], wherein an X-ray powder diffraction pattern of the crystal has a peak at diffraction angles (2θ) of
   4.9°, 9.4°, 9.9°, and 15.2° (± 0.2°),
   4.9°, 9.4°, 9.9°, and 15.8° (± 0.2°),
   4.9°, 9.4°, 9.9°, and 18.9° (± 0.2°),
   4.9°, 9.4°, 9.9°, and 22.6° (± 0.2°),
   4.9°, 9.4°, 15.2°, and 15.8° (± 0.2°),
   4.9°, 9.4°, 15.2°, and 18.9° (± 0.2°),
   4.9°, 9.4°, 15.2°, and 22.6° (± 0.2°),
   4.9°, 9.4°, 15.8°, and 18.9° (± 0.2°),
   4.9°, 9.4°, 15.8°, and 22.6° (± 0.2°),
   4.9°, 9.4°, 18.9°, and 22.6° (± 0.2°),
   4.9°, 9.9°, 15.2°, and 15.8° (± 0.2°),
   4.9°, 9.9°, 15.2°, and 18.9° (± 0.2°),
   4.9°, 9.9°, 15.2°, and 22.6° (± 0.2°),
   4.9°, 9.9°, 15.8°, and 18.9° (± 0.2°),
   4.9°, 9.9°, 15.8°, and 22.6° (± 0.2°),
   4.9°, 9.9°, 18.9°, and 22.6° (± 0.2°),
   4.9°, 15.2°, 15.8°, and 18.9° (± 0.2°),
   4.9°, 15.2°, 15.8°, and 22.6° (± 0.2°),
   4.9°, 15.2°, 18.9°, and 22.6° (± 0.2°),
   4.9°, 15.8°, 18.9°, and 22.6° (± 0.2°),
   9.4°, 9.9°, 15.2°, and 15.8° (± 0.2°),
   9.4°, 9.9°, 15.2°, and 18.9° (± 0.2°),
   9.4°, 9.9°, 15.2°, and 22.6° (± 0.2°),
   9.4°, 9.9°, 15.8°, and 18.9° (± 0.2°),
   9.4°, 9.9°, 15.8°, and 22.6° (± 0.2°),
   9.4°, 9.9°, 18.9°, and 22.6° (± 0.2°),
   9.4°, 15.2°, 15.8°, and 18.9° (± 0.2°),
   9.4°, 15.2°, 15.8°, and 22.6° (± 0.2°),
   9.4°, 15.2°, 18.9°, and 22.6° (± 0.2°),
   9.4°, 15.8°, 18.9°, and 22.6° (± 0.2°),
   9.9°, 15.2°, 15.8°, and 18.9° (± 0.2°),
   9.9°, 15.2°, 15.8°, and 22.6° (± 0.2°),
   9.9°, 15.2°, 18.9°, and 22.6° (± 0.2°),
   9.9°, 15.8°, 18.9°, and 22.6° (± 0.2°), or
   15.2°, 15.8°, 18.9° and 22.6° (± 0.2°).
[2-43] The pharmaceutical formulation according to any of [2-33] to [2-42], wherein an X-ray powder diffraction pattern of the crystal has a peak at at least five diffraction angles (2θ) selected from the group consisting of 4.9°, 9.4°, 9.9°, 15.2°, 15.8°, 18.9° and 22.6° (± 0.2°).
[2-44] The pharmaceutical formulation according to any of [2-33] to [2-43] wherein an X-ray powder diffraction pattern of the crystal has a peak at diffraction angles (2θ) of
   4.9°, 9.4°, 9.9°, 15.2°, and 15.8° (± 0.2°),
   4.9°, 9.4°, 9.9°, 15.2°, and 18.9° (± 0.2°),
   4.9°, 9.4°, 9.9°, 15.2°, and 22.6° (± 0.2°),
   4.9°, 9.4°, 9.9°, 15.8°, and 18.9° (± 0.2°),
   4.9°, 9.4°, 9.9°, 15.8°, and 22.6° (± 0.2°),
   4.9°, 9.4°, 9.9°, 18.9°, and 22.6° (± 0.2°),
   4.9°, 9.4°, 15.2°, 15.8°, and 18.9° (± 0.2°),
   4.9°, 9.4°, 15.2°, 15.8°, and 22.6° (± 0.2°),
   4.9°, 9.4°, 15.2°, 18.9°, and 22.6° (± 0.2°),
   4.9°, 9.4°, 15.8°, 18.9°, and 22.6° (± 0.2°),
   4.9°, 9.9°, 15.2°, 15.8°, and 18.9° (± 0.2°),
   4.9°, 9.9°, 15.2°, 15.8°, and 22.6° (± 0.2°),
   4.9°, 9.9°, 15.2°, 18.9°, and 22.6° (± 0.2°),
   4.9°, 9.9°, 15.8°, 18.9°, and 22.6° (± 0.2°),
   4.9°, 15.2°, 15.8°, 18.9°, and 22.6° (± 0.2°),
   9.4°, 9.9°, 15.2°, 15.8°, and 18.9° (± 0.2°),
   9.4°, 9.9°, 15.2°, 15.8°, and 22.6° (± 0.2°),
   9.4°, 9.9°, 15.2°, 18.9°, and 22.6° (± 0.2°),
   9.4°, 9.9°, 15.8°, 18.9°, and 22.6° (± 0.2°),
   9.4°, 15.2°, 15.8°, 18.9°, and 22.6° (± 0.2°), or
   9.9°, 15.2°, 15.8°, 18.9° and 22.6° (± 0.2°).
[2-45] The pharmaceutical formulation according to any of [2-33] to [2-44], wherein an X-ray powder diffraction pattern of the crystal has a peak at at least six diffraction angles (2θ) selected from the group consisting of 4.9°, 9.4°, 9.9°, 15.2°, 15.8°, 18.9° and 22.6° (± 0.2°).
[2-46] The pharmaceutical formulation according to [2-45] wherein an X-ray powder diffraction pattern of the crystal has a peak at diffraction angles (2θ) of
   4.9°, 9.4°, 9.9°, 15.2°, 15.8°, and 18.9° (± 0.2°),
   4.9°, 9.4°, 9.9°, 15.2°, 15.8°, and 22.6° (± 0.2°),
   4.9°, 9.4°, 9.9°, 15.2°, 18.9°, and 22.6° (± 0.2°),
   4.9°, 9.4°, 9.9°, 15.8°, 18.9°, and 22.6° (± 0.2°),
   4.9°, 9.4°, 15.2°, 15.8°, 18.9°, and 22.6° (± 0.2°),
   4.9°, 9.9°, 15.2°, 15.8°, 18.9°, and 22.6° (± 0.2°), or
   9.4°, 9.9°, 15.2°, 15.8°, 18.9° and 22.6° (± 0.2°).
[2-47] The pharmaceutical formulation according to any of [2-33] to [2-46], wherein an X-ray powder diffraction pattern of the crystal has a peak at diffraction angles (2θ) of 4.9°, 9.4°, 9.9°, 15.2°, 15.8°, 18.9° and 22.6° (± 0.2°).
[2-48] The pharmaceutical formulation according to any of [2-33] to [2-47], wherein a volume-based particle size of the crystal of the p-toluenesulfonate of the compound represented by formula 1 has d₁₀ of less than 5.76 µm, d₅₀ of less than 8.81 µm, or d₉₀ of less than 13.08 µm.
[2-49] The pharmaceutical formulation according to any of [2-33] to [2-48], wherein a volume-based particle size of the crystal of the p-toluenesulfonate of the compound represented by formula 1 has d₁₀ of 5.00 µm or less, d₅₀ of 8.00 µm or less, or d₉₀ of 12.00 µm or less.
[2-50] The pharmaceutical formulation according to any of [2-33] to [2-49], wherein a volume-based particle size of the crystal of the p-toluenesulfonate of the compound represented by formula 1 has d₁₀ of 4.00 µm or less, d₅₀ of 6.00 µm or less, or d₉₀ of 11.00 µm or less.
[2-51] The pharmaceutical formulation according to any of [2-33] to [2-50], wherein a volume-based particle size of the crystal of the p-toluenesulfonate of the compound represented by formula 1 has d₁₀ of 3.00 µm or less, d₅₀ of 5.00 µm or less, or d₉₀ of 8.00 µm or less.
[2-52] The pharmaceutical formulation according to any of [2-33] to [2-51], wherein a volume-based particle size of the crystal of the p-toluenesulfonate of the compound represented by formula 1 has d₁₀ of 1.00 µm or more and less than 5.76 µm, d₅₀ of 2.30 µm or more and less than 8.81 µm, or d₉₀ of 4.30 µm or more and less than 13.08 µm.
[2-53] The pharmaceutical formulation according to any of [2-33] to [2-52], wherein a volume-based particle size of the crystal of the p-toluenesulfonate of the compound represented by formula 1 has d₁₀ of 1.20 µm or more and less than 5.76 µm, d₅₀ of 2.40 µm or more and less than 8.81 µm, or d₉₀ of 4.50 µm or more and less than 13.08 µm.
[2-54] The pharmaceutical formulation according to any of [2-33] to [2-53], wherein a volume-based particle size of the crystal of the p-toluenesulfonate of the compound represented by formula 1 has d₁₀ of 1.30 µm or more and less than 5.76 µm, d₅₀ of 2.50 µm or more and less than 8.81 µm, or d₉₀ of 4.70 µm or more and less than 13.08 µm.
[2-55] The pharmaceutical formulation according to any of [2-33] to [2-54], wherein a volume-based particle size of the crystal of the p-toluenesulfonate of the compound represented by formula 1 has d₁₀ of less than 5.76 µm, d₅₀ of less than 8.81 µm, and d₉₀ of less than 13.08 µm.
[2-56] The pharmaceutical formulation according to any of [2-33] to [2-55], wherein a volume-based particle size of the crystal of the p-toluenesulfonate of the compound represented by formula 1 has d₁₀ of 5.00 µm or less, d₅₀ of 8.00 µm or less, and d₉₀ of 12.00 µm or less.
[2-57] The pharmaceutical formulation according to any of [2-33] to [2-56], wherein a volume-based particle size of the crystal of the p-toluenesulfonate of the compound represented by formula 1 has d₁₀ of 4.00 µm or less, d₅₀ of 6.00 µm or less, and d₉₀ of 11.00 µm or less.
[2-58] The pharmaceutical formulation according to any of [2-33] to [2-57], wherein a volume-based particle size of the crystal of the p-toluenesulfonate of the compound represented by formula 1 has d₁₀ of 3.00 µm or less, d₅₀ of 5.00 µm or less, and d₉₀ of 8.00 µm or less.
[2-59] The pharmaceutical formulation according to any of [2-33] to [2-58], wherein a volume-based particle size of the crystal of the p-toluenesulfonate of the compound represented by formula 1 has d₁₀ of 1.00 µm or more and less than 5.76 µm, d₅₀ of 2.30 µm or more and less than 8.81 µm, and d₉₀ of 4.30 µm or more and less than 13.08 µm.
[2-60] The pharmaceutical formulation according to any of [2-33] to [2-59], wherein a volume-based particle size of the crystal of the p-toluenesulfonate of the compound represented by formula 1 has d₁₀ of 1.20 µm or more and less than 5.76 µm, d₅₀ of 2.40 µm or more and less than 8.81 µm, and d₉₀ of 4.50 µm or more and less than 13.08 µm.
[2-61] The pharmaceutical formulation according to any of [2-33] to [2-60], wherein a volume-based particle size of the crystal of the p-toluenesulfonate of the compound represented by formula 1 has d₁₀ of 1.30 µm or more and less than 5.76 µm, d₅₀ of 2.50 µm or more and less than 8.81 µm, and d₉₀ of 4.70 µm or more and less than 13.08 µm.
[2-62] The pharmaceutical formulation according to any of [2-1] to [2-61], wherein the pharmaceutical formulation is in a form of a capsule containing a p-toluenesulfonate of the compound, which is an active ingredient, as a type 1 crystal, and further containing mannitol, croscarmellose sodium, and sodium stearyl fumarate.
[2-63] The pharmaceutical formulation according to any of [2-1] to [2-62], wherein the pharmaceutical formulation is a powder containing a p-toluenesulfonate of the compound, which is an active ingredient, as a type 1 crystal, and further containing mannitol, croscarmellose sodium, and sodium stearyl fumarate.
[2-64] The pharmaceutical formulation according to [2-63], wherein the powder is used as a filler of a capsule.
[3-1] A method for producing the pharmaceutical composition according to any of [1-1] to [1-59] or [1-64], including pulverizing a p-toluenesulfonate of the compound represented by formula 1: 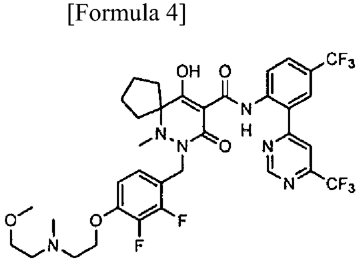 .
[3-2] A method for producing the pharmaceutical formulation according to any of [1-60] to [1-63] or [2-1] to [2-64], including pulverizing a p-toluenesulfonate of the compound represented by formula 1: 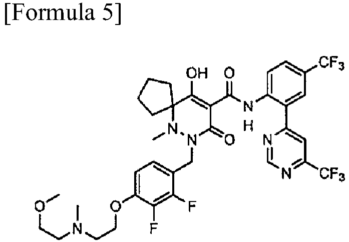 .
[3-3] The method according to [3-1] or [3-2], further including mixing the p-toluenesulfonate of the compound represented by formula 1, an excipient, a disintegrant, and a lubricant to obtain a mixture.
[3-4] The method according to [3-2] or [3-3], further including filling the mixture into a capsule shell.
[4-1] A method for producing a pharmaceutical composition, including mixing a p-toluenesulfonate of the compound represented by formula 1 and a lubricant to obtain a mixture.
[4-2] A method for producing a pharmaceutical composition, including mixing a p-toluenesulfonate of the compound represented by formula 1, an excipient, a disintegrant, and a lubricant to obtain a mixture.
[4-3] The production method according to [4-1] or [4-2], wherein a content of the lubricant is in a range of 5.3 wt% or more and 15 wt% or less based on a total of the pharmaceutical composition.
[4-4] The production method according to any of [4-1] to [4-3], wherein a content of the lubricant is in a range of 7.0 wt% or more and 11 wt% or less based on the total of the pharmaceutical composition.
[4-5] The production method according to any of [4-2] to [4-4], wherein a content of the excipient is in a range of 21 wt% or more and 72 wt% or less based on the total of the pharmaceutical composition.
[4-6] The production method according to any of [4-2] to [4-5], wherein a content of the excipient is in a range of 27 wt% or more and 66 wt% or less based on the total of the pharmaceutical composition.
[4-7] The production method according to any of [4-2] to [4-6], wherein a content of the disintegrant is in a range of 10 wt% or more and 30 wt% or less based on the total of the pharmaceutical composition.
[4-8] The production method according to any of [4-2] to [4-7], wherein a content of the disintegrant is in a range of 18 wt% or more and 22 wt% or less based on the total of the pharmaceutical composition.
[4-9] The production method according to any of [4-1] to [4-8], wherein sodium stearyl fumarate, magnesium stearate, calcium stearate, or talc is contained as the lubricant.
[4-10] The production method according to any of [4-1] to [4-9], wherein sodium stearyl fumarate is contained as the lubricant.
[4-11] The production method according to any of [4-2] to [4-10], wherein mannitol or lactose hydrate is contained as the excipient.
[4-12] The production method according to any of [4-2] to [4-11], wherein mannitol is contained as the excipient.
[4-13] The production method according to any of [4-2] to [4-12], wherein croscarmellose sodium, carmellose sodium, carmellose calcium, or hydroxypropyl cellulose is contained as the disintegrant.
[4-14] The production method according to any of [4-2] to [4-13], wherein croscarmellose sodium or carmellose calcium is contained as the disintegrant.
[4-15] The production method according to any of [4-1] to [4-14], wherein a content of the compound represented by formula 1 or a salt thereof or a solvate of these is in a range of 1 wt% or more and 65 wt% or less based on the total of the pharmaceutical composition.
[4-16] The production method according to any of [4-1] to [4-15], wherein a content of the p-toluenesulfonate of the compound represented by formula 1 is in a range of 8 wt% or more and 45 wt% or less based on the total of the pharmaceutical composition.
[4-17] The production method according to any of [4-1] to [4-16], wherein the p-toluenesulfonate of the compound represented by formula 1 is a type 1 crystal.
[4-18] The production method according to any of [4-1] to [4-17], including pulverizing the p-toluenesulfonate of the compound represented by formula 1.
[4-19] The production method according to [4-17] or [4-18], wherein a volume-based particle size of the crystal of the p-toluenesulfonate of the compound represented by formula 1 has d₁₀ of less than 5.76 µm, d₅₀ of less than 8.81 µm, or d₉₀ of less than 13.08 µm.
[4-20] The production method according to any of [4-17] to [4-19], wherein a volume-based particle size of the crystal of the p-toluenesulfonate of the compound represented by formula 1 has d₁₀ of 4.00 µm or less, d₅₀ of 6.00 µm or less, or d₉₀ of 11.00 µm or less.
[4-21] The production method according to any of [4-17] to [4-20], wherein a volume-based particle size of the crystal of the p-toluenesulfonate of the compound represented by formula 1 has d₁₀ of less than 5.76 µm, d₅₀ of less than 8.81 µm, and d₉₀ of less than 13.08 µm.
[4-22] The production method according to any of [4-17] to [4-21], wherein a volume-based particle size of the crystal of the p-toluenesulfonate of the compound represented by formula 1 has d₁₀ of 4.00 µm or less, d₅₀ of 6.00 µm or less, and d₉₀ of 11.00 µm or less.
[4-23] The production method according to any of [4-1] to [4-22], wherein the pharmaceutical composition is a powder containing a p-toluenesulfonate of the compound represented by formula 1, which is an active ingredient, as a type 1 crystal, and further containing mannitol, croscarmellose sodium, and sodium stearyl fumarate.
[4-24] The method according to any of [4-1] to [4-23], further including filling the mixture into a capsule shell.

### ADVANTAGEOUS EFFECTS OF INVENTION

The present invention enables compound I, which has a strong inhibitory action on NaPi-IIb, PiT-1, and PiT-2 but is hardly soluble in water, to have improved solubility in the gastrointestinal tract by formulation. Furthermore, the present invention enables stable production of formulations having uniformity of or above a certain level in the production of formulations of compound I.

### BRIEF DESCRIPTION OF DRAWINGS

Figure 1 is a graph showing the results of the dissolution test of compound I regarding the formulations of Examples 1 (F59) and 2 (F61).
Figure 2 is a graph showing the results of the dissolution test of compound I regarding the formulations of Example 3 (F50), Example 4 (F51), Example 5 (F52) and Example 2 (F66).
Figure 3 is a graph showing the results of the dissolution test of compound I regarding the capsule formulation of Example 12 (R4L02).
Figure 4 is a graph showing the results of the dissolution test of compound I regarding the capsule formulation of Example 13 (R4L05).
Figure 5 is a diagram showing the measurement results of the particle size distribution of compound I.
Figure 6 is a diagram showing the measurement results of the particle size distribution of compound I.
Figure 7 is a diagram showing the measurement results of the particle size distribution of compound I.
Figure 8 is a graph showing the results of the dissolution test of compound I regarding the formulation using a pulverized product of the drug substance of compound I.

### DESCRIPTION OF EMBODIMENTS

The present specification relates to a formulation containing 7-[[2,3-difluoro-4-[2-[2-methoxyethyl(methyl)amino]ethoxy]phenyl]methyl]-10-hydroxy-6-methyl-8-oxo-N-[4-(trifluoromethyl)-2-[6-(trifluoromethyl)pyrimidin-4-yl]phenyl]-6,7-diazaspiro[4,5]deca-9-ene-9-carboxamide or a salt thereof or a solvate of these as a drug substance, for example, a formulation containing a p-toluenesulfonate of the compound (compound I) as a drug substance.

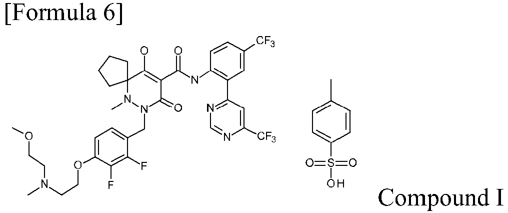

The compound I mentioned above can be produced by converting a compound obtained by the method described in Example 14 of Patent Literature 6 to a p-toluenesulfonate. More specifically, compound I can be produced by the method described in Non Patent Literature 9.

As referred herein to a salt of the compound of formula 1, the salt is not particularly limited as long as it is a pharmaceutically acceptable salt formed by an acid or base acceptable for ingestion along with administration of a medicament. Examples thereof include organic acid salts, inorganic acid salts, organic base salts, or inorganic base salts, specifically, carboxylates such as acetate, citrate, malate, tartrate, and succinate; sulfonates such as methanesulfonates, benzenesulfonates, and p-toluenesulfonates; hydrochlorides; hydrobromides; hydroiodides; sulfates; phosphates; nitrates; or alkali metal salts such as sodium salts and potassium salts; alkaline earth metal salts such as magnesium salts and calcium salts; and ammonium salts such as alkylammonium salts, dialkylammonium salts, trialkylamonium salts, and tetraalkylammonium salts. These salts are produced by, for example, contacting the compound represented by formula 1 with an acid or base that can be used in the production of a medicament.

As referred herein to a solvate, the solvate refers to one in which a compound and a solvent together form a molecular aggregate, and is not particularly limited as long as it is a pharmaceutically acceptable solvate formed by a solvent acceptable for ingestion along with administration of a medicament. Examples of the solvate include a hydrate, an alcohol solvate (such as an ethanol solvate, a methanol solvate, a 1-propanol solvate, a 2-propanol solvate), and not only a solvate formed with a single solvent such as dimethyl sulfoxide, but also a solvate formed with a plurality of solvents per one molecule of the compound or a solvate formed with a plurality of types of solvents per one molecule of the compound.

In the production of pharmaceutical compositions or pharmaceutical formulations containing compound I, powdered crystals of compound I can be used. For example, powdered crystals obtained by the method described in Non Patent Literature 9, specifically powdered crystals obtained by precipitating from a solution in which acetone, ethyl acetate, ethanol, or heptane, and a mixture of these solvents is used as a solvent, can be used.

The compound used herein may contain an isotopic atom at a non-natural ratio in one or more atoms that constitute the compounds. The compound having any atom replaced with another isotopic atom having the same atomic number (the number of protons) and a different mass number (sum of the numbers of protons and neutrons), and thereby having an isotopic abundance different from the isotopic abundance in nature, that is, a compound labeled with an isotopic atom, is also included in the present invention. Examples of the isotopic element contained in the compounds herein include a hydrogen atom, a carbon atom, a nitrogen atom, an oxygen atom, a phosphorus atom, a sulfur atom, a fluorine atom, and a chlorine atom, and they include ²H, ³H; ¹³C, ¹⁴C; ¹⁵N; ¹⁷O, ¹⁸O; ³²P; ³⁵S; ¹⁸F; ³⁶Cl; and the like, respectively. The compound labeled with an isotopic atom is useful as a therapeutic or prophylactic agent, a research reagent (e.g., assay reagent), and a diagnostic agent (e.g., in vivo imaging diagnostic agent). For the compounds as used herein, all the compounds containing any proportions of radioactive or non-radioactive isotopic elements are encompassed within the scope of the present invention. The compound labeled with an isotopic atom can be produced in a manner similar to methods for producing unlabeled compounds by using a reagent or a solvent containing a corresponding isotopic atom.

The "pharmaceutical composition" herein refers to a preparation of a pharmacologically active compound herein with a medium such as an additive, a carrier, or a diluent that is generally acceptable in the art for delivery to a mammal such as a human.

The "formulation" and "pharmaceutical formulation" herein refers to a product in which the active ingredient used in a medicament has been processed into an optimum shape or property in accordance with the method of use and the purpose of use. Depending on the method of use, the purpose of use, and the nature of the active ingredient, additives may be added if necessary. Examples of the type of the pharmaceutical formulations, that is "dosage form", include liquid pharmaceutical formulations (liquids) such as an injection, a suspension, an emulsion, an eye drop; and solid pharmaceutical formulations (solid formulations) such as a tablet, a powder, a fine granule, a granule, a coated tablet, a capsule, a dry syrup, a lozenge, and a suppository, but are not limited to these.

The "formulation" and "pharmaceutical formulation" herein is produced by a well-known method using additives such as an excipient, a disintegrant, a lubricant, a binder, a lubricant colorant, a flavoring agent, a stabilizer, an emulsifier, an absorption enhancer, a surfactant, a pH adjuster, a preservative, and an antioxidant.

The "solid formulation" herein refers to a dosage form such as a tablet, a powder, a fine granule, a granule, a coated tablet, a capsule, a dry syrup, a lozenge, or a suppository. The dosage form refers to a form itself of a shaped medicament or the like. As the solid formulation according to the present specification, a capsule or a tablet is preferred, and a capsule is more preferred. These formulations are not particularly limited as long as they have normal components and normal shape and size commonly used in the formulation field, and their form and the like is not particularly limited.

The "excipient" herein is an additive added for molding, bulking, diluting or the like of a solid formulation. Examples thereof include, but are not particularly limited to, sugar (e.g., lactose, lactose hydrate, fructose, glucose), sugar alcohol (e.g., mannitol), starch (corn starch, potato starch, wheat starch, rice starch, partially pregelatinized starch, pregelatinized starch), cellulose (e.g., crystalline cellulose), and inorganic salts (e.g., calcium silicate, calcium hydrogen phosphate anhydrous, precipitated calcium carbonate). More specific examples include corn starch, potato starch, wheat starch, rice starch, partially pregelatinized starch, pregelatinized starch, lactose hydrate, fructose, glucose, mannitol, calcium hydrogen phosphate anhydrous, crystalline cellulose, and precipitated calcium carbonate, and preferably include mannitol, lactose, and crystalline cellulose.

The "disintegrant" herein is an additive added for promoting the disintegration of a solid formulation administered into the body and increasing the rate of release of an active ingredient from the formulation. Examples thereof include, but are not particularly limited to, croscarmellose sodium, carmellose sodium, hydroxypropyl cellulose, carmellose, carmellose calcium, methylcellulose, crystalline cellulose, sodium lauryl sulfate, povidone, and polysorbate, and preferably include croscarmellose sodium and carmellose sodium.

The "lubricant" herein is an additive added for increasing fluidity of a mixture used in the manufacturing process of the solid formulation so that the mixture containing an active ingredient is mixed uniformly, or for facilitating the compression molding of the solid formulation. Examples thereof include, but are not particularly limited to, magnesium stearate, calcium stearate, talc, a sucrose fatty acid ester, sodium stearyl fumarate, and a hardened oil. Preferred examples thereof include sodium stearyl fumarate, magnesium stearate, and a hardened oil, more preferably sodium stearyl fumarate and magnesium stearate, and most preferably sodium stearyl fumarate.

As used herein, "wt%" refers to the ratio of the weight of a particular substance to the weight of the total components. When weight% is used to describe the amount of a component in a pharmaceutical composition, in a pharmaceutical formulation, or in a solid formulation, it means the ratio of the weight of a particular component to the weight of all components contained in the pharmaceutical composition, pharmaceutical formulation, or solid formulation. The weight of packaging containers, such as ampoules, plastic bottles, or boxes, capsule shells in capsules, or the like, which are used to prevent dispersion of pharmaceutical compositions, pharmaceutical formulations, solid formulations, or the like during handling, such as when transporting, storing, or administering thereof, are not included in the weight of the total of all components, unless specifically noted.

When the content of an additive in the pharmaceutical composition is expressed by wt% based on the total of the pharmaceutical composition herein, the wt% represents the ratio of the weight of the additive to the weight of the total of the pharmaceutical composition consisting of an active ingredient and the additive added to the active ingredient. When the pharmaceutical formulation is encapsulated in a container in advance, the weight of the container is not included in the weight of the total of the pharmaceutical formulation. Examples of the pharmaceutical formulation encapsulated in a container include not only a pharmaceutical formulation which is used by opening the container and then administering the content, such as a pill enclosed in a bottle, a tablet enclosed in an aluminum sheet, or an injection enclosed in a syringe, but also a pharmaceutical formulation in which a filler (a mixture of an active ingredient and an additive, also referred to as a capsule filler, filler, filler powder or bulk powder) is encapsulated in a container having a shape of capsule, and the capsule encapsulating the filler therein is used for administration, such as a capsule. In one aspect of the present invention, the pharmaceutical composition is a filler that is a content of a capsule. The filler is preferably a powdered formulation, and the powdered formulation may be used as a pharmaceutical formulation encapsulated in a capsule shell, which may be administered as a capsule.

When the pharmaceutical composition herein is used in the form of a capsule, the capsule shell can be used one commonly used in capsules, such as those described in Japanese Pharmacopoeia (17th edition or 18th edition). The type of capsule shell is not particularly limited, and those commonly used in the art can be used. Examples of the type of capsule shell include a hard capsule shell and a soft capsule shell. Preferred examples of the raw material that can be used in the hard capsule shell include gelatin, hydroxypropyl methylcellulose, pullulan, and a mixture thereof, and a gelatin capsule shell is preferably used. Preferred examples of the raw material that can be used in the soft capsule shell include gelatin, starch, carrageenan, agar, glycerol, sorbitol, and a mixture thereof, and a gelatin capsule shell is preferably used.

When the content of the active ingredient is expressed by wt% based on the total of the pharmaceutical composition herein, the wt% represents the ratio of the weight, i.e., the content, of the active ingredient to the weight of the total of the pharmaceutical composition consisting of an active ingredient and the additive added to the active ingredient.

Regarding the content of compound I, which is the active ingredient, based on the total of the pharmaceutical composition herein, the lower limit is, for example, 1 wt%, 3 wt%, 5 wt%, 7 wt%, or 8 wt% and the upper limit is, for example, 65 wt%, 60 wt%, 55 wt%, 50 wt%, or 45 wt%. The lower limit is preferably 8 wt%, and the upper limit is preferably 45 wt%. The preferred range of the content is, for example, a range of 1 wt% or more and 65 wt% or less, a range of 3 wt% or more and 60 wt% or less, a range of 5 wt% or more and 55 wt% or less, or a range of 7 wt% or more and 50 wt% or less, and more preferably a range of 8 wt% or more and 45 wt% or less.

As the additive herein, an excipient can be used. When the content of the excipient is expressed by wt% based on the total of the pharmaceutical composition herein, the wt% represents the ratio of the weight, i.e., the content, of the excipient to the weight of the total of the pharmaceutical composition consisting of an active ingredient and the additive added to the active ingredient.

Regarding the content of the excipient based on the total of the pharmaceutical composition herein, the lower limit is, for example, 21 wt%, 23 wt%, 25 wt%, 26 wt%, or 27 wt% and the upper limit is, for example, 72 wt%, 70 wt%, 68 wt%, 67 wt%, or 66 wt%. The lower limit is preferably 27 wt%, and the upper limit is preferably 66 wt%. The preferred range of the content is, for example, a range of 21 wt% or more and 72 wt% or less, a range of 23 wt% or more and 70 wt% or less, a range of 25 wt% or more and 68 wt% or less, or a range of 26 wt% or more and 67 wt% or less, and more preferably a range of 27 wt% or more and 66 wt% or less. Preferred examples of the excipient include mannitol.

As the additive herein, a disintegrant can be used. When the content of the disintegrant is expressed by wt% based on the total of the pharmaceutical composition herein, the wt% represents the ratio of the weight, i.e., the content, of the disintegrant to the weight of the total of the pharmaceutical composition consisting of an active ingredient and the additive added to the active ingredient.

Regarding the content of the disintegrant based on the total of the pharmaceutical composition herein, the lower limit is, for example, 10 wt%, 12 wt%, 14 wt%, 16 wt%, or 18 wt% and the upper limit is, for example, 30 wt%, 28 wt%, 26 wt%, 24 wt%, or 22 wt%. The lower limit is preferably 18 wt%, and the upper limit is preferably 22 wt%. The preferred range of the content is, for example, a range of 10 wt% or more and 30 wt% or less, a range of 12 wt% or more and 28 wt% or less, a range of 14 wt% or more and 26 wt% or less, or a range of 16 wt% or more and 24 wt% or less, and more preferably a range of 18 wt% or more and 22 wt% or less. Preferred examples of the disintegrant include croscarmellose sodium and/or carmellose sodium.

As the additive herein, a lubricant can be used. When the content of the lubricant is expressed by wt% based on the total of the pharmaceutical composition herein, the wt% represents the ratio of the weight, i.e., the content, of the lubricant to the weight of the total of the pharmaceutical composition consisting of an active ingredient and the additive added to the active ingredient.

Regarding the content of the lubricant based on the total of the pharmaceutical composition herein, the lower limit is, for example, 5.3 wt%, 5.6 wt%, 6.0 wt%, 6.3 wt%, 6.5 wt%, 6.8 wt%, or 7.0 wt% and the upper limit is, for example, 15 wt%, 14 wt%, 13 wt%, 12 wt%, 11 wt%, or 10 wt%. The lower limit is preferably 7.0 wt%, and the upper limit is preferably 10 wt%. The preferred range of the content is, for example, a range of 5.3 wt% or more and 15 wt% or less, a range of 5.6 wt% or more and 14 wt% or less, or a range of 6.0 wt% or more and 13 wt% or less, more preferably a range of 6.3 wt% or more and 12 wt% or less, a range of 6.5 wt% or more and 11 wt% or less, a range of 6.8 wt% or more and 11 wt% or less, or a range of 7.0 wt% or more and 11 wt% or less, and most preferably a range of 7.0 wt% or more and 10 wt% or less.

Regarding the ratio of the lubricant to 1 part by weight of the compound I herein, the lower limit is, for example, 0.10 parts by weight, 0.13 parts by weight, or 0.15 parts by weight, and the upper limit is, for example, 0.90 parts by weight, 0.87 parts by weight, or 0.85 parts by weight. The lower limit is preferably 0.15 parts by weight, and the upper limit is preferably 0.85 parts by weight. The preferred range of the ratio is, for example, a range of 0.10 parts by weight or more and 0.90 parts by weight or less, or a range of 0.13 parts by weight or more and 0.87 parts by weight or less, and more preferably a range of 0.15 parts by weight or more and 0.85 parts by weight or less. Preferred examples of the lubricant include sodium stearyl fumarate or magnesium stearate, and more preferred examples of the lubricant include sodium stearyl fumarate.

The additive contained in pharmaceutical compositions and pharmaceutical formulations containing compound I is not particularly limited as long as it is a formulation used in the pharmaceutical composition and pharmaceutical formulations. Examples of the additive include an excipient, a disintegrant, a lubricant, a binder, a lubricant colorant, a flavoring agent, a stabilizer, an emulsifier, an absorption enhancer, a surfactant, a pH adjusting agent, a preservative, and an antioxidant. The type and amount of the additives used in the pharmaceutical composition and the pharmaceutical formulation are optionally selected depending on the physical and chemical properties of the active ingredient contained in the pharmaceutical composition and the pharmaceutical formulation, and they can be optimized for use.

The "volume-based particle size" herein refers to the particle size distribution value weighted by volume among the particle sizes of the powder, and represents the particle size in a volume that accounts for a predetermined ratio in the powder sample. It may also be expressed as volume average particle size such as volume-based particle size d₁₀ (abbreviated as d₁₀), volume-based particle size d₅₀ (abbreviated as d₅₀), or volume-based particle size d₉₀ (abbreviated as d₉₀). d₁₀, d₅₀, or d₉₀ refer to the particle size of a point corresponding to 10%, 50%, or 90% respectively of the sample volume when the cumulative frequency distribution curve of particle size is determined based on the total volume of the particle as 100%. A person skilled in the art can perform the measurement by using marketed equipment for particle size measurement in accordance with the manual for the equipment. For example, a volume-based particle size measurement with a laser diffraction particle size distribution measurement device can be performed by dispersing a powder to be measured in a solvent (dispersion medium) that disperses the powder, irradiating the dispersion medium in which the powder is dispersed with laser light, and measuring the change in laser light diffraction over time as the dispersion state of the powder. The measurement can also be performed, for example, in accordance with a conventional method such as "Particle Size Determination" described in Japanese Pharmacopoeia (17th edition or 18th edition). For example, the volume-based particle size of compound I can also be determined according to the following method.
(1) SPAN80 (about 3 g) is weighed, and n-hexane (3000 mL) is added to prepare a solution of 0.1 wt% SPAN80 in n-hexane.
(2) About 50 mg of compound I is weighed, 2000 mL of the solution of 0.1 wt% SPAN80 in n-hexane is added and stirred to prepare a saturated solution.
(3) Insoluble materials of the saturated solution are filtered off with a 0.45 µm disposable decompression filter, then the filtrate is used as a dispersion medium.
(4) Approximately 30 mg of compound I is precisely weighed, and the dispersion medium (2 mL) is added to prepare a sample to be measured.
(5) The test by laser diffraction method is performed three times under the following conditions, and the average value is taken as the particle size.

Refractive index: sample: 1.6900, imaginary part: 0.0100, dispersion medium (hexane): 1.3760
Repeats: 15
Base of particle size: volume
Number of data imports: 5000
Transmittance (appropriate range) Red semiconductor laser: 90 to 80%
Blue light emitting diode: 90 to 70% LA-950V2 manufactured by HORIBA, Ltd. can be used as the measuring equipment.

In one aspect of the volume-based particle size of the crystal of compound I herein, the volume-based particle size is preferably a fine particle size that can increase the dissolution rate to achieve the active ingredient of the solid to be dissolved in the body and absorbed efficiently. It is also preferred that the solid of the active ingredient has a certain level of particle size distribution so that the content of the active ingredient is uniform in the pharmaceutical compositions and the pharmaceutical formulations.

Regarding the volume-based particle size of the crystal of compound I herein, the upper limit of d₁₀ is, for example, less than 4.00 µm, 5.00 µm, or 5.76 µm. The lower limit of d₁₀ is, for example, 1.30 µm, 1.20 µm, or 1.00 µm. The upper limit is preferably less than 5.76 µm, and the lower limit is preferably 1.00 µm. The range of d₁₀ is, for example, a range of 1.00 µm or more and less than 5.76 µm, or a range of 1.20 µm or more and 5.00 µm or less, and more preferably, a range of 1.30 µm or more and 4.00 µm or less.

Regarding the volume-based particle size of the crystal of compound I herein, the upper limit of d₅₀ is, for example, less than 5.00 µm, 8.00 µm, or 8.81 µm. The lower limit of d₅₀ is, for example, 2.50 µm, 2.40 µm, or 2.30 µm. The upper limit is preferably less than 8.81 µm, and the lower limit is preferably 2.30 µm. The range of d₅₀ is, for example, a range of 2.30 µm or more and less than 8.81 µm, or a range of 2.40 µm or more and 8.00 µm or less, and more preferably, a range of 2.50 µm or more and 5.00 µm or less.

Regarding the volume-based particle size of the crystal of compound I herein, the upper limit of d₉₀ is, for example, less than 8.00 µm, 10.00 µm, 12.00 µm, or 13.08 µm. The lower limit of d₉₀ is, for example, 4.70 µm, 4.60 µm, 4.50 µm, or 4.30 µm. The upper limit is preferably less than 13.08 µm, and the lower limit is preferably 2.30 µm. The range of d₉₀ is, for example, a range of 4.30 µm or more and less than 13.08 µm, a range of 4.50 µm or more and 12.00 µm or less, or a range of 4.60 µm or more and 10.00 µm or less, and more preferably, a range of 4.70 µm or more and 8.00 µm or less.

Regarding the volume-based particle size of the crystal of compound I herein, d₁₀ is less than 5.76 µm, d₅₀ is less than 8.81 µm, or d₉₀ is less than 13.08 µm.

Regarding the volume-based particle size of the crystal of compound I herein, d₁₀ is a range of 1.00 µm or more and 5.76 µm or less, a range of 1.30 µm or more and 4.00 µm or less, or a range of 1.20 µm or more and 5.00 µm or less, d₅₀ is a range of 2.30 µm or more and less than 8.81 µm, a range of 2.40 µm or more and 8.00 µm or less, or a range of 2.50 µm or more and 5.00 µm or less, or d₉₀ is a range of 4.30 µm or more and less than 13.08 µm, a range of 4.50 µm or more and 12.00 µm or less, a range of 4.60 µm or more and 10.00 µm or less, or a range of 4.70 µm or more and 8.00 µm or less.

Regarding the volume-based particle size of the crystal of compound I herein, d₁₀ is less than 5.76 µm, d₅₀ is less than 8.81 µm, and d₉₀ is less than 13.08 µm.

Regarding the volume-based particle size of the crystal of compound I herein, d₁₀ is a range of 1.00 µm or more and less than 5.76 µm, a range of 1.30 µm or more and 4.00 µm or less, or a range of 1.20 µm or more and 5.00 µm or less, d₅₀ is a range of 2.30 µm or more and less than 8.81 µm, a range of 2.40 µm or more and 8.00 µm or less, or a range of 2.50 µm or more and 5.00 µm or less, and d₉₀ is a range of 4.30 µm or more and less than 13.08 µm, a range of 4.50 µm or more and 12.00 µm or less, a range of 4.60 µm or more and 10.00 µm or less, or a range of 4.70 µm or more and 8.00 µm or less.

The "X-ray powder diffraction" herein refers to a numerical value which can be determined using an X-ray diffraction phenomenon that is used for identification and structural analysis of a crystalline substance, and the value is specific to a certain crystal. This value is typically represented by one or more 2θ values. A person skilled in the art can perform the measurement by using marketed equipment for X-ray powder diffraction measurement in accordance with the manual for the equipment. More specifically, a sample to be measured is irradiated with an X-ray of CuKα, and a diffraction X-ray is measured with respect to an incident X-ray. In this way, the 2θ value can be measured. For example, the measurement can be performed in accordance with a conventional method such as "X-ray Powder Diffractometry" described in Japanese Pharmacopoeia (17th edition or 18th edition).

The peak value (2θ value) in an X-ray powder diffraction spectrum may have some margin of error depending on measurement equipment or measurement conditions such as peak reading conditions. The peak value herein may have a measurement error in the range of ± 0.2°, or about ± 0.2°, e.g., ± 0.5°. In Japanese Pharmacopoeia (17th edition or 18th edition), it is described that the diffraction angle 2θ in the same crystal form usually matches within a range of ± 0.2°. Thus, not only crystals whose peak diffraction angles in X-ray powder diffraction match perfectly, but also crystals whose peak diffraction angles match within an error of about ± 0.2° are included in the present invention.

The term "(± 0.2°)" described at the end of listed diffraction angles 2θ in the written diffraction angles 2θ herein means that the range of ± 0.2° with respect to each described value is acceptable for all the listed diffraction angles 2θ. The same applies to the term "± 0.5°".

The "X-ray powder diffraction pattern" herein means a pattern obtained by plotting a peak value by diffraction (also referred to as diffraction angle, 2θ value, or 2θ) obtained by the measurement of X-ray powder diffraction spectrum, and the intensity thereof on the abscissa and the ordinate, respectively. A person skilled in the art can perform the plotting by using marketed equipment for X-ray powder diffraction measurement in accordance with the manual for the equipment. The analysis by X-ray powder diffraction can also be performed, for example, in accordance with a conventional method such as a method described in "X-ray Powder Diffractometry" in Japanese Pharmacopoeia (17th edition or 18th edition).

The "type 1 crystal" of compound I herein is one of the crystal of the p-toluenesulfonate of the compound represented by formula (I) (compound I), and it is indicated that the type 1 crystal of compound I has peaks at diffraction angles (2θ) around 4.9°, 9.4°, 9.9°, 15.2°, 15.8°, 18.9° and 22.6° in an X-ray powder diffraction pattern (Non Patent Literature 9).

In one aspect, the X-ray powder diffraction pattern of the "type 1 crystal" of compound I herein has a peak at at least one diffraction angle (2θ) selected from the group consisting of 4.9°, 9.4°, 9.9°, 15.2°, 15.8°, 18.9° and 22.6° (± 0.2°).

In one aspect, the X-ray powder diffraction pattern of the "type 1 crystal" of compound I herein has peaks at at least two diffraction angles (2θ) selected from the group consisting of 4.9°, 9.4°, 9.9°, 15.2°, 15.8°, 18.9° and 22.6° (± 0.2°). Preferable examples of the two diffraction angles include 4.9° and 9.4° (± 0.2°), 4.9° and 15.2° (± 0.2°), 4.9° and 18.9° (± 0.2°), 4.9° and 22.6° (± 0.2°), 15.2° and 18.9° (± 0.2°), or 15.2° and 22.6° (± 0.2°).

In one aspect, the X-ray powder diffraction pattern of the "type 1 crystal" of compound I herein has peaks at at least three diffraction angles (2θ) selected from the group consisting of 4.9°, 9.4°, 9.9°, 15.2°, 15.8°, 18.9° and 22.6° (± 0.2°). Preferable examples of the combination of three diffraction angles include 4.9°, 9.4° and 15.2° (± 0.2°), 4.9°, 15.2° and 18.9° (± 0.2°), 4.9°, 15.2° and 22.6° (± 0.2°), 9.4°, 15.2° and 18.9° (± 0.2°), or 15.2°, 18.9° and 22.6° (± 0.2°).

In one aspect, the X-ray powder diffraction pattern of the "type 1 crystal" of compound I herein has peaks at at least four diffraction angles (2θ) selected from the group consisting of 4.9°, 9.4°, 9.9°, 15.2°, 15.8°, 18.9° and 22.6° (± 0.2°) in 2θ values in the X-ray powder diffraction. Preferable examples of the combination of four diffraction angles include 4.9°, 9.4°, 15.2° and 18.9° (± 0.2°), 4.9°, 9.4°, 15.2° and 22.6° (± 0.2°), 4.9°, 15.2°, 18.9° and 22.6° (± 0.2°), 9.4°, 15.2°, 18.9° and 22.6° (± 0.2°), or 15.2°, 15.8°, 18.9° and 22.6° (± 0.2°).

In one aspect, the X-ray powder diffraction pattern of the "type 1 crystal" of compound I herein has peaks at at least five diffraction angles (2θ) selected from the group consisting of 4.9°, 9.4°, 9.9°, 15.2°, 15.8°, 18.9° and 22.6° (± 0.2°). Preferable examples of the combination of diffraction angles of peaks include 4.9°, 9.4°, 15.2°, 18.9° and 22.6° (± 0.2°) or 4.9°, 9.9°, 15.2°, 18.9° and 22.6° (± 0.2°).

In one aspect, the X-ray powder diffraction pattern of the "type 1 crystal" of compound I herein has peaks at at least six diffraction angles (2θ) selected from the group consisting of 4.9°, 9.4°, 9.9°, 15.2°, 15.8°, 18.9° and 22.6° (± 0.2°). Preferable examples of the combination of six diffraction angles include 4.9°, 9.4°, 9.9°, 15.8°, 18.9° and 22.6° (± 0.2°) or 4.9°, 9.4°, 15.2°, 15.8°, 18.9° and 22.6° (± 0.2°).

The X-ray powder diffraction pattern of the "type 1 crystal" of compound I herein has peaks at all diffraction angles (2θ) of 4.9°, 9.4°, 9.9°, 15.2°, 15.8°, 18.9° and 22.6° (± 0.2°) in 2θ values in the X-ray powder diffraction.

The "thermogravimetric analysis" herein refers to a method for thermally analyzing a change in physical and chemical properties of a sample, which is analysis means for measuring a weight change caused by heating a sample. A person skilled in the art can perform the measurement by using marketed equipment for thermogravimetric analysis measurement in accordance with the manual for the equipment.

The "differential thermal analysis" herein refers to analysis means for detecting and measuring heat generation or heat absorption caused by heating a sample. A person skilled in the art can perform the measurement by using marketed equipment for differential thermal analysis measurement in accordance with the manual for the equipment. The use of "thermogravimetric analysis" and/or "differential thermal analysis" enables acquisition of information on a physical phenomenon such as sublimation, melting, solidification, condensation, evaporation, decomposition, adsorption or desorption of the sample. When multiple samples are measured by "thermogravimetric analysis" and/or "differential thermal analysis" and they have, for example, the same melting point of the solid, the measurement results can be data suggesting that these multiple samples have the same crystalline form. Also, when, for example, a sample among them shows a high melting point of the solid, the measurement results can be data that the sample is more stable to heat compared to a sample having a low melting point. The thermogravimetric analysis and differential thermal analysis can be performed, for example, in accordance with a conventional method such as a method described in "Thermal Analysis" in Japanese Pharmacopoeia (17th edition).

It is indicated that, in thermogravimetric analysis herein, compound I shows peaks associated with melting at 112.6°C (extrapolation point) and 126.6°C (peak top) (Non Patent Literature 9).

The "dissolution test" herein can be performed, for example, in accordance with a method described in "Dissolution Test" in Japanese Pharmacopoeia (17th edition or 18th edition). As the test fluid used in the dissolution test, the 1st or 2nd fluid for dissolution test of Japanese Pharmacopoeia (17th edition or 18th edition), or the solution with additives added thereto can be used. Examples of the additives include a surfactant such as Tween 80. The dissolution test is performed by a paddle method. In one aspect of the present invention, the pharmaceutical composition has a drug dissolution rate of 75% or more, 80% or more, or 85% or more after 15 minutes in a dissolution test (paddle method, 75 revolutions per minute, 37°C) using 900 mL of a test fluid of pH 1.2.

The "content uniformity test" herein can be performed, for example, in accordance with a method of content uniformity test described in Japanese Pharmacopoeia (17th edition or 18th edition). In one aspect of the present invention, the pharmaceutical composition is a hard capsule, and when it is subjected to the content uniformity test described in Japanese Pharmacopoeia (17th edition or 18th edition) and a determination value is calculated, the determination value confirms to the determination criteria (the determination value does not exceed L1% (15.0%)).

The pharmaceutical composition and pharmaceutical formulation herein can be produced by a general production method after a step of pulverizing the p-toluenesulfonate of the compound represented by formula 1.

The step of pulverizing the p-toluenesulfonate of the compound represented by formula 1 can be performed, for example, by pulverizing for one cycle in a pulverizing chamber using a jet mill pulverizer in accordance with the manual for the pulverizer.

The pharmaceutical composition and pharmaceutical formulation herein can also be produced by a general production method after a step of mixing the p-toluenesulfonate of the compound represented by formula 1, an excipient, a disintegrant, and a lubricant. The step of mixing the p-toluenesulfonate of the compound represented by formula 1, an excipient, a disintegrant, and a lubricant can be carried out, for example, by mixing the p-toluenesulfonate of the compound represented by formula 1, the excipient, the disintegrant, and the lubricant for 90 minutes at a rotational speed of 60 rpm in a mixer.

Further, the pharmaceutical composition and pharmaceutical formulation herein can be produced by a production method including a step of pulverizing the p-toluenesulfonate of the compound represented by formula 1, or a step of mixing the p-toluenesulfonate of the compound represented by formula 1, an excipient, a disintegrant, and a lubricant, and then further including a step of filling a mixture of the p-toluenesulfonate of the compound represented by formula 1, the excipient, the disintegrant, and the lubricant into a capsule shell. The step of mixing the p-toluenesulfonate of the compound represented by formula 1, an excipient, a disintegrant, and a lubricant can be carried out, for example, by filling the mixture into a gelatin capsule (No. 1) with a capsule filling machine.

The compound represented by formula 1 or a salt thereof or a solvate of these has a strong inhibitory action on NaPi-IIb, PiT-1, and PiT-2, excellent stability in the body, and excellent absorbability from the gastrointestinal tract, and is useful as a preventive agent or a therapeutic agent, particularly as a therapeutic agent, for a chronic disease such as hyperphosphatemia. The compound represented by formula 1 or a salt thereof or a solvate of these are useful as a preventive agent or a therapeutic agent for various diseases caused by an increase in phosphorus concentration in the body, such as hyperphosphatemia, secondary hyperparathyroidism, chronic kidney disease, chronic renal failure, and arteriosclerosis with vascular calcification.

When the composition of the present invention is used as an inhibitor of NaPi-IIb, PiT-1, and PiT-2, or a preventive agent or a therapeutic agent for hyperphosphatemia, secondary hyperparathyroidism, chronic kidney disease, chronic renal failure and arteriosclerosis with vascular calcification, the dosage of the compound represented by formula 1 of the active ingredient or a salt thereof or a solvate of these may vary depending on the condition of the disease, the age, the weight, the presence or absence of other drugs, and the like. For example, when the composition of the present invention is administered to a patient as an oral agent, the amount of the active ingredient per dose is, for example, 50 to 500 mg, 50 to 200 mg, preferably 100 to 300 mg, in terms of the compound represented by formula 1 or a salt thereof or a solvate of these. The number of doses per day is, for example, one to three times. That is, it is administered, for example, every 24 hours, every 12 hours, or every 8 hours, such as before meals, between meals, after meals, or before bedtime.

The meaning of the term "and/or" herein includes any combination in which "and" and "or" are appropriately combined. Specifically, for example, the term "A, B and/or C" includes the following seven variations; (i) A, (ii) B, (iii) C, (iv) A and B, (v) A and C, (vi) B and C, and (vii) A, B and C.

The abbreviations used herein are listed below.
AV: acceptable value
cCMC-Na: croscarmellose sodium
CMC-Ca: carmellose calcium
Lac: lactose monohydrate
MAN: mannitol
MCC: microcrystalline cellulose
Mg-st: magnesium stearate
rpm: revolutions per minute
SSF: sodium stearyl fumarate

### [Examples]

The contents of the present invention are further described in detail by the following Examples, but the present invention is not limited to these contents. All starting materials and reagents can be obtained from commercial suppliers, or synthesized using known methods. The capsule shell that can be used herein is a capsule shell that is commonly used in capsule formulations. In this example, Pearitol 300DC (manufactured by Roquette Frères) was used for mannitol, ND-2HS (manufactured by Asahi Kasei Chemicals Corp.) was used for cCMC-Na, E.C.G-505 (manufactured by GOTOKU CHEMICAL COMPANY LTD.) was used for CMC-Ca, Parteck LUB MST (manufactured by Merck KGaA) was used for Mg-st., and PRUV (manufactured by JRS PHARMA LP) was used for SSF. Pharmatose 200M (manufactured by DMV-Fonterra Excipients GmbH & Co. KG) was used for lactose. The capsule shell used was Japanese Pharmacopoeia capsule No. 1 with Qualicaps gelatin (manufactured by Qualicaps).

### Method of pulverizing drug substance

The drug substance used was a powdered crystal of compound I produced by the method described in International Publication No. WO 2014/142273 and Japan Institute for Promoting Invention and Innovation, Journal of technical disclosure, Technique No. 2017-501666. The drug substance (1345.4 mg, product of lot number SGL1407010) was pulverized for one cycle in a pulverizing chamber using a jet mill pulverizer (MC-ONE, manufactured by Dietrich Engineering Consultants sa) in accordance with the manual for the pulverizer to obtain a pulverized product (1029.0 mg). The same pulverizing process was performed for products of lot number RGW1503010 and lot number RGW1601010 as shown in Table 7.

### Production of filler powder

Compound I and the additive were weighed and added to a MIGHTY VIAL (No.7, clear, Maruemu Corporation) so that the total weight of the filler powder was 10 g. The contents were mixed by rotating MIGHTY VIAL for 60 minutes or more at a rotational speed of 100 rpm with MIX-ROTER VMR-5 (AS ONE Corporation) to obtain a sample.

For a control lactose triturated powder (compound I : lactose monohydrate = 1 : 9 (006/Lac)), compound I and lactose were weighed and added to a MIGHTY VIAL (No.7, clear, Maruemu Corporation) so that the total weight of the filler powder was 10 g. The contents were mixed by rotating MIGHTY VIAL for 60 minutes or more at a rotational speed of 100 rpm with MIX-ROTER VMR-5 (AS ONE Corporation) to obtain a sample.

**[Table 1]**

| Table 1: Composition of filler powder | | | | | | |
|---|---|---|---|---|---|---|
| | Content (wt%) | | | | | |
| Example No. | 1 (F59) | 2 (F61) | 3 (F50) | 4 (F51) | 5 (F52) | 6 (F66) |
| Compound I | 41.2 | 41.2 | 41.2 | 41.2 | 41.2 | 41.2 |
| MAN | 31.8 | 31.8 | 58.3 | 48.3 | 38.3 | 28.3 |
| cCMC-Na | 20 | | | 10 | 20 | 30 |
| CMC-Ca | | 20 | | | | |
| Mg-st. | | | 0.5 | 0.5 | 0.5 | 0.5 |
| SSF | 7 | 7 | | | | |
| Total | 100 | 100 | 100 | 100 | 100 | 100 |

When SSF was used, no filler powder was found on the glass wall, suggesting that the mixing was well performed.

### Dissolution Test

The dissolution test of the prepared filler powder was performed by the paddle method under the following conditions.
Amount of solution: 900 mL
Temperature of solution: 37°C ± 0.2°C
Dissolution test fluid: a solution obtained by dissolving 2.0 g of sodium chloride in 7.0 mL of hydrochloric acid and water to 1000 mL, and further adding 0.05% Tween 80 (a solution containing 0.05% Tween 80 in the 1st fluid for dissolution test of Japanese Pharmacopoeia (17th edition))
Sampling: fractionation method
Stirring device: paddle
Stirring speed: 75 rpm
Occurrence of precipitate: Yes
UV Detection wavelength: 293 nm

### Measurement method of dissolution rate by ultraviolet-visible absorbance test method

Absorbance of the dissolution test fluid at a wavelength of 293 nm was measured by an online UV dissolution test system (manufactured by Shimadzu Corporation). The dissolution rate of capsule formulations each containing 25 mg or 100 mg of compound I was calculated based on the absorbance of the standard solutions prepared so that the concentrations of p-toluenesulfonate of the compound represented by formula 1 (compound I) were 34 µg/mL and 136 µg/mL in an aqueous 50% acetonitrile solution.

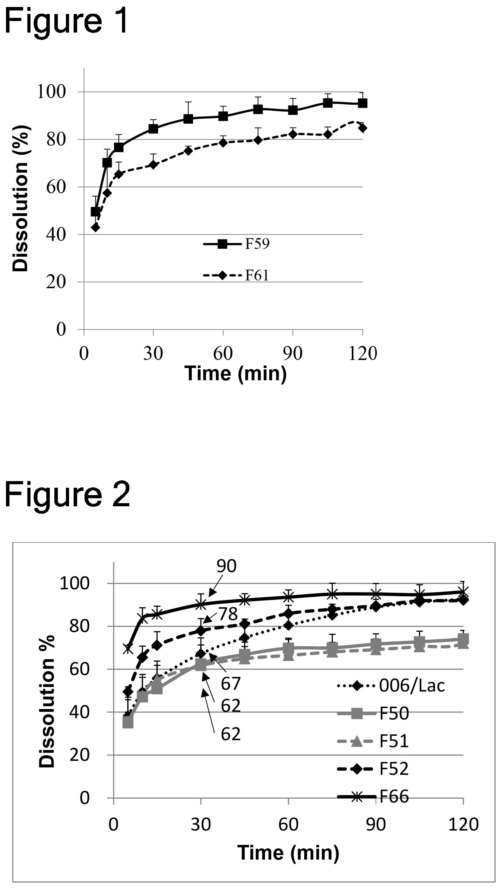

The filler powder (300 mg) containing compound I described in Examples 1 to 6 (Table 1) was filled into a gelatin capsule (No. 1), and the dissolution rate of compound I dissolved in the dissolution test fluid was measured by the ultraviolet-visible absorbance test method. The results are shown in Figures 1 and 2. Dissolution property of formulations (capsule, tablet, or the like) contributes to the evaluation of the absorbability of a medicament that is a content of the formulations. In addition, since the formulation disintegrates and dissolves in an aqueous solution, an evaluation of disintegration property is a simple evaluation of the dissolution property.

Figure 1 shows the comparison of dissolution property of compound I when cCMC-Na (Example 1 (F59)) and CMC-Ca (Example 2 (F61) were used as the disintegrant. It was shown that the filler powder containing cCMC-Na (Example 1 (F59)) had excellent dissolution property of compound I.

Figure 2 shows the comparison of dissolution property of compound I when the formulation ratio of cCMC-Na was changed from 0% to 30%. The dissolution property of compound I when the ratio of cCMC-Na in the filler powder was 20% or more (Examples 5 (F52) and 6 (F66)) surpassed that of the control lactose triturated powder (compound I : lactose monohydrate = 1 : 9 (006/Lac)), indicating excellent dissolution property. It was shown that the preferred content of cCMC-Na in the filler powder was 20 wt% or more.

### Adhesion test

The adhesion test was performed by placing a filler powder in a glass container, and visually observing the adhesion to the inner wall of the container. Details of the sample preparation are shown below. Compound I and the additive were weighed and added to a MIGHTY VIAL (No.7, clear, Maruemu Corporation) so that the total weight of the filler powder was 10 g. The contents were mixed by rotating MIGHTY VIAL for 60 minutes or more at a rotational speed of 100 rpm with MIX-ROTER VMR-5 (AS ONE Corporation) to obtain a sample.

SSF was changed to 0.5 to 9 wt%, and the adhesion to the glass vial at the mixing of compound I and the additive was compared. The adhesion to the glass wall was observed visually.

**[Table 2]**

| Table 2: Composition of filler powder | | | | | |
|---|---|---|---|---|---|
| | Content (wt%) | | | | |
| Example No. | 7 (F67) | 8 (F55) | 9 (F58) | 10 (F59) | 11 (F68) |
| Compound I | 41.2 | 41.2 | 41.2 | 41.2 | 41.2 |
| MAN | 38.3 | 35.8 | 33.8 | 31.8 | 29.8 |
| cCMC-Na | 20 | 20 | 20 | 20 | 20 |
| SSF | 0.5 | 3 | 5 | 7 | 9 |
| Total | 100 | 100 | 100 | 100 | 100 |
| Adhesion to glass wall | Yes | Yes | Yes | No | No |
| | | | | | |

When the filler powder contained 7 wt% or more SSF, lumps of the filler powder were not adhered to the glass wall. Suppression of the adhesion is important to ensure the content uniformity of the formulation. It was suggested that the preferred content added of SSF is above 5 wt%, for example, 6 wt% or more, or 7 wt% or more.

### Production of capsule formulation

Capsule formulations each containing 25 mg or 100 mg of the active ingredient in terms of a free base (a compound represented by formula 1) was produced by mixing compound I with the additive so that the total weight of the filler powder of the capsule formulation was 3600 g, in accordance with the methods below. The filler powder was produced by mixing at a rotational speed of 60 rpm for 90 minutes using a mixer (Rocking mixer, model number RMC-10(S)MC, manufactured by Aichi Electric Co., Ltd.). The filler powder was filled into a gelatin capsule (No. 1) with a capsule filling machine (Capsule filling apparatus, model number: MODEL300A, manufactured by Acuraks Inc.) to produce a 25 mg capsule formulation (Example 12 (R4L02)) and a 100 mg capsule formulation (Example 13 (R4L05)).

**[Table 3]**

| Table 3: Composition of capsule | | | | | | |
|---|---|---|---|---|---|---|
| | Composition (wt%) | | | Composition (mg) | | |
| Component/amount | Content | 25 mg | 100 mg | Content | 25 mg | 100 mg |
| Compound I | 0 | 8.49 | 33.97 | 0 | 30.6 | 122.3 |
| MAN | 73 | 64.51 | 39.03 | 262.8 | 232.2 | 140.5 |
| cCMC-Na | 20 | 20 | 20 | 72 | 72 | 72 |
| SSF | 7 | 7 | 7 | 25.2 | 25.2 | 25.2 |
| Gelatin capsule shell | - | - | - | - | - | - |
| Total | 100 | 100 | 100 | 360 | 360 | 360 |

### Dissolution test of capsule

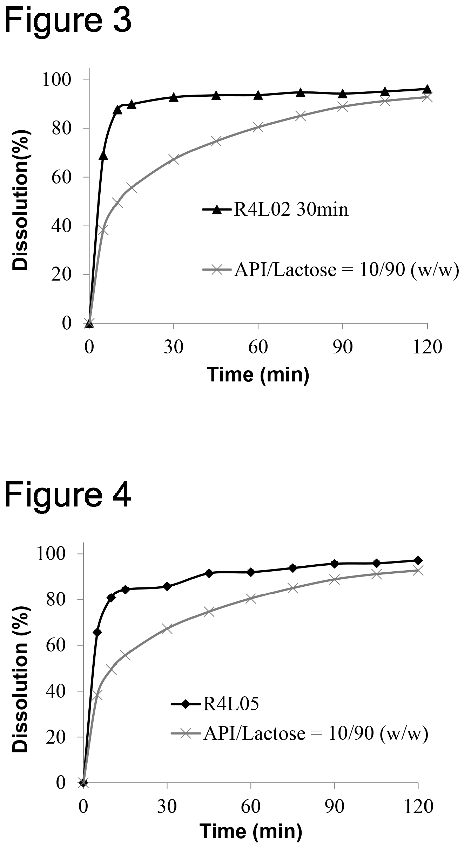

Dissolution tests of the 25 mg capsule formulation (Example 12) and the 100 mg capsule formulation (Example 13) were performed by the same dissolution test method as described above. The results are shown in Figures 3 and 4.

The dissolution property of these capsule formulations surpassed that of a capsule formulation using the control lactose triturated powder (compound I : lactose monohydrate = 1 : 9 (006/Lac)), indicating excellent dissolution property.

### Content uniformity test of capsule

The content value of the content uniformity test was measured by HPLC, and averaged to determine based on Japanese Pharmacopoeia (17th edition). Details are shown below.

Ten samples were prepared for each filler powder of the 25 mg capsule formulation (Example 12 (R4L02)) and the 100 mg capsule formulation (Example 13 (R4L05)) according to the following method. When the obtained value exceeded the maximum allowable limit value (L1%) of the determination value calculated by calculation of the determination values, the same test was performed again with changing the number of samples to 20, and the determination value was calculated. X mL of a dissolution solvent (a solvent obtained by weighing 1000 of water and 1000 mL of acetonitrile with a measuring cylinder, and after mixing, adding 1 mL of trifluoroacetic acid and then mixing the mixture (a mixed solution of water/acetonitrile/trifluoroacetic acid (1000 : 1000 : 1)) was placed in a Y mL measuring flask (Table 4). The flask was shaken at 100 revolutions per minute for 30 minutes with a shaker (product of AS ONE Corporation, Model ASCM-01) to disintegrate the sample.

**[Table 4]**

| Table 4: Measuring flask and amount of solvent used | | |
|---|---|---|
| Capsule formulation | XmL | YmL |
| 25 mg capsule | 20 | 50 |
| 100 mg capsule | 80 | 200 |

The dissolution solvent was added to the measuring flask, and the mixture was further mixed, and sonication was performed for 15 minutes (the measuring flask was shaken about every 5 minutes from the start of sonication). Then, the dissolution solvent was added to fill up. The mixture was filtered with a membrane filter (product of ADVANTEC CO.,LTD., Model 25HP020AN, DISMIC-25HP PTFE 0.20 µm hydrophilic), then 1 mL of the initial filtrate was removed off, and the following filtrate was used as a sample solution.

In addition, a standard solution was prepared by the following method.

Compound I (12 mg), separately produced as a standard substance, was precisely weighed, and the dissolution solvent was added to dissolve it to prepare an exact 20 mL of solution. When the dissolution was insufficient, it was dissolved by sonication (about 1 minute). The amount of the solution was accurately adjusted with the dissolution solvent to prepare a standard solution.

The measurement conditions are as follows.
System: ACQUITY UPLC H-Class
Equipment Name: Quaternary Solvent Manager (H-class QSM) (Waters Corporation)
   Sample Manager-FTN (H-class SM-FTN) (Waters Corporation)
   Sample Organizer (H-class SO) (Waters Corporation)
   Column Heater (H-class CH) (Waters Corporation)
Detector: TUV Detector (TUV) (Waters Corporation)
Column: Kinetex XB-C18, 4.6 mm × 50 mm, 2.6 µm (Phenomenex)
Mobile phase: A) 0.05% TFA/water, B) 0.05% TFA/acetonitrile
Flow rate: 1.0 mL/min
Detection wavelength: 225 nm
Column temperature: 30°C
Injection amount: 3.0 µL

**[Table 5]**

| Table 5: Gradient | | |
|---|---|---|
| Time (min) | Mobile phase A (vol%) | Mobile phase B (vol%) |
| 0 | 70 | 30 |
| 6.0 | 5 | 95 |
| 7.5 | 5 | 95 |
| 7.6 | 70 | 30 |

The results are shown in Table 6.

**[Table 6]**

| Table 6: Content uniformity | | |
|---|---|---|
| Example No. | 12 (R4L02) | 13 (R4L05) |
| Average content (wt%, n=10) | 98.1 | 99 |
| SD (%, n=10) | 2.3 | 2.5 |
| RSD (%, n=10) | 2.4 | 2.6 |
| AV (n=10) | 5.9 | 5.6 |

From the test results, it was confirmed that the content uniformity of the 25 mg capsule formulation (R4L02) and the 100 mg capsule formulation (R4L05) met the target value of the determination value (AV) (the maximum allowable limit value (L1) of the determination value to be 15 or less), indicating excellent content uniformity.

### Particle size distribution of compound I

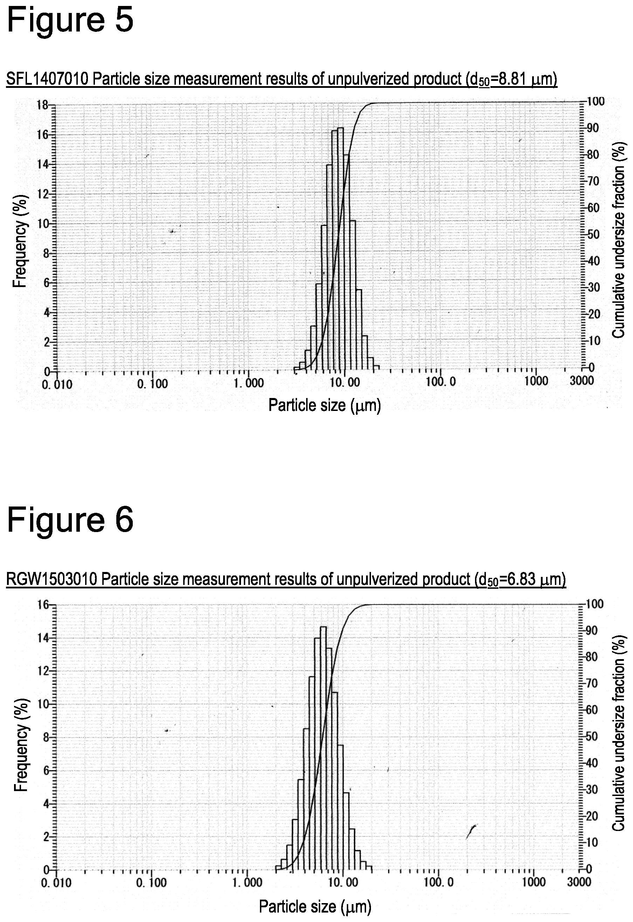

The particle size distributions of a pulverized product, which was obtained by the pulverizing method of the crystalline powder of compound I described above, and an unpulverized product were measured by laser diffraction method, and the volume-based particle size d₁₀, d₅₀, and d₉₀ (µm) were calculated. The sample preparation of the light scattering method was performed as described below.
(1) SPAN80 (about 3 g) was weighed, and n-hexane (3000 mL) was added to prepare a solution of 0.1 wt% SPAN80 in n-hexane.
(2) About 50 mg of the unpulverized product was weighed, 2000 mL of the solution of 0.1 wt% SPAN80 in n-hexane was added and the mixture was stirred to prepare a saturated solution.
(3) Insoluble materials of the saturated solution were filtered off with a 0.45 µm disposable decompression filter (Millicup-LH, PTFE, 0.45 µm, manufactured by Millipore, model SJLHM4710), then the filtrate was used as a dispersion medium.
(4) Approximately 30 mg of the sample was precisely weighed, and the dispersion medium (2 mL) was added to prepare a sample to be measured.
(5) The test was performed three times and the average value was taken.
   The measurement conditions are described below.
   Equipment Name: LA-950V2 manufactured by HORIBA, Ltd.
   Refractive index: sample: 1.6900, imaginary part: 0.0100, dispersion medium (hexane): 1.3760
   Repeats: 15
   Base of particle size: volume
   Number of data imports: 5000
   Transmittance (appropriate range) Red semiconductor laser: 90 to 80%
   Blue light emitting diode: 90 to 70% The measurement results are shown in Table 7 and Figures 5 to 7 below.

**[Table 7]**

| Table 7: Measurement results of volume-based particle size (µm) | | | | | |
|---|---|---|---|---|---|
| Lot number | SFL1407010 | | RGW1503010 | | RGW1601010 |
| | Before pulverizing | After pulverizing | Before pulverizing | After pulverizing | After pulverizing |
| d₁₀ | 5.76 | 1.9 | 4.13 | 2.0 | 1.5 |
| d₅₀ | 8.81 | 3.5 | 6.83 | 3.4 | 2.6 |
| d₉₀ | 13.08 | 5.9 | 11.4 | 5.8 | 4.8 |

### Effect of particle size of drug substance on dissolution property

44 mg of lactose monohydrate (200 mesh) was mixed with 6 mg of the unpulverized or pulverized products, and the dissolution test in fasted state simulated intestinal fluid at 37°C was performed by the paddle method under the following conditions with measurement by UPLC. The dissolution test was performed at n = 4.

### <Dissolution test conditions>

Solution amount: 50 mL
Temperature: 37°C
Solution: fasted state simulated intestinal fluid (FaSSIF)
Sampling: VK8000 dissolution sampling station (Varian Medical Systems, Inc.)
Device: VK7010 dissolution station (Varian Medical Systems, Inc.)
Rotational speed: 50 rpm

### <UPLC conditions>

System: Acquity UPLC (Waters Corporation)
Column: Acquity UPLC BEH Shield RP18, 1.7 µm, 2.1 × 50.0 mm (Waters Corporation)
Mobile phase: A) 0.05% TFA/water, B) 0.05% TFA/acetonitrile
Flow rate: 1.0 mL/min
Detection wavelength: 260 nm
Column temperature: 40°C
Injection amount: 5 µL

**[Table 8]**

| Table 8: Gradient | | |
|---|---|---|
| Time (min) | Mobile phase A (vol%) | Mobile phase B (vol%) |
| 0 | 95 | 5 |
| 0.95 | 2 | 98 |
| 1.40 | 2 | 98 |
| 1.41 | 95 | 5 |
| 1.50 | 95 | 5 |

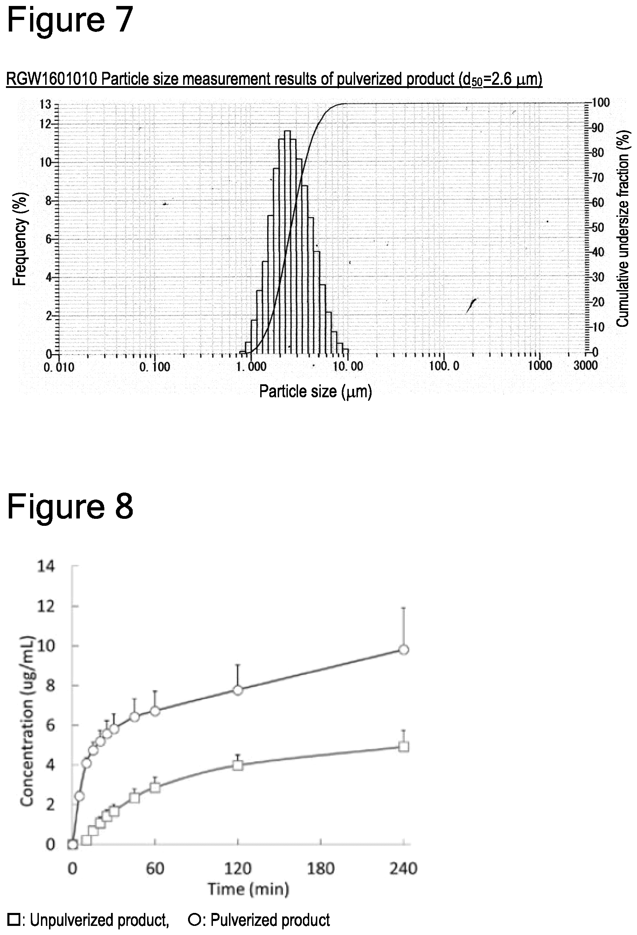

The results are shown in Figure 8. It was confirmed that excellent dissolution property was achieved when a pulverized product of the drug substance was used compared to when a non-pulverized product was used.

### INDUSTRIAL APPLICABILITY

The present invention can provide a pharmaceutical composition and a production method of the same for treating hyperphosphatemia in a patient having a renal dysfunction such as a chronic kidney disease (CKD) or an end-stage kidney disease (ESKD).
The present invention is characterized by the following preferred embodiments:
1. A pharmaceutical composition comprising:
   a compound represented by formula 1: 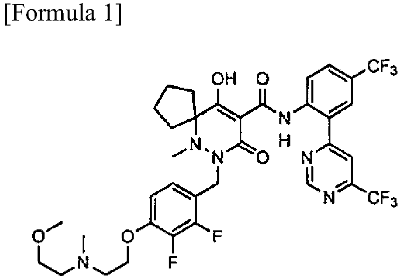 or a salt thereof or a solvate of these and a lubricant.
2. The pharmaceutical composition according to item 1, further comprising an excipient and a disintegrant.
3. The pharmaceutical composition according to item 1 or 2, wherein a content of the lubricant is 0.5 wt% or more based on a total of the pharmaceutical composition.
4. The pharmaceutical composition according to any one of items 1 to 3, wherein a content of the excipient is 25 wt% or more based on the total of the pharmaceutical composition.
5. The pharmaceutical composition according to any one of items 1 to 4, wherein a content of the disintegrant is 10 wt% or more based on the total of the pharmaceutical composition.
6. The pharmaceutical composition according to any one of items 1 to 5, wherein, as the lubricant, at least one lubricant selected from the group consisting of sodium stearyl fumarate, zinc stearate, aluminum stearate, calcium stearate, magnesium stearate, talc, and a sucrose fatty acid ester is contained.
7. The pharmaceutical composition according to any one of items 1 to 6, wherein, as the excipient, at least one excipient selected from the group consisting of mannitol, lactose hydrate, fructose, glucose, sorbitol, corn starch, potato starch, wheat starch, and rice starch is contained.
8. The pharmaceutical composition according to any one of items 1 to 7, wherein, as the disintegrant, at least one disintegrant selected from the group consisting of croscarmellose sodium, carmellose sodium, hydroxypropyl cellulose, carmellose, carmellose calcium, methylcellulose, crystalline cellulose, sodium lauryl sulfate, povidone, and polysorbate is contained.
9. The pharmaceutical composition according to any one of items 1 to 8, wherein a content of the compound represented by formula 1 or a salt thereof or a solvate of these is in a range of 5 wt% or more and 50 wt% or less based on the total of the pharmaceutical composition.
10. The pharmaceutical composition according to any one of items 1 to 9, wherein the compound represented by formula 1 or a salt thereof or a solvate of these is a p-toluenesulfonate of the compound represented by formula 1.
11. The pharmaceutical composition according to any one of items 1 to 10, wherein the p-toluenesulfonate of the compound represented by formula 1 is a type 1 crystal.
12. The pharmaceutical composition according to item 10 or 11, wherein a volume-based particle size of the p-toluenesulfonate of the compound represented by formula 1 has d₁₀ of less than 5.76 µm, d₅₀ of less than 8.81 µm, or d₉₀ of less than 13.08 µm.
13. A pharmaceutical formulation comprising the pharmaceutical composition according to any one of items 1 to 12.
14. The pharmaceutical formulation according to item 13, wherein the pharmaceutical formulation is selected from the group consisting of a powdered formulation, a powder, a granule, a tablet, and a capsule.
15. The pharmaceutical formulation according to item 13 or 14, wherein the pharmaceutical formulation is a capsule.
16. The pharmaceutical composition according to any one of items 1 to 12, wherein the pharmaceutical composition is in a form of a capsule containing a p-toluenesulfonate of the compound represented by formula 1, which is an active ingredient, as a type 1 crystal, and further containing mannitol, croscarmellose sodium, and sodium stearyl fumarate.
17. A method for producing a pharmaceutical composition containing a p-toluenesulfonate salt of a compound represented by formula 1: 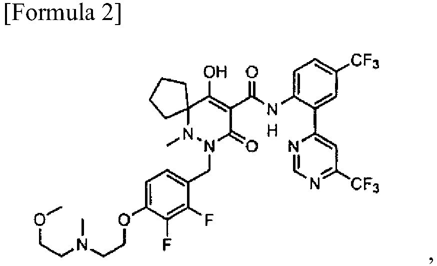 an excipient, a disintegrant, and a lubricant.

## Claims

1. A pharmaceutical composition comprising:
a type 1 crystal of a p-toluenesulfonate salt of the compound represented by formula 1: 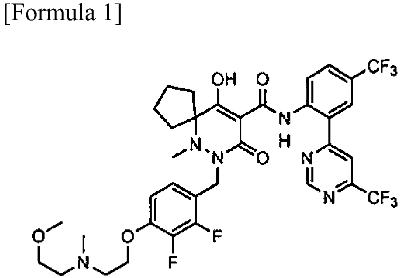 and a lubricant.

2. The pharmaceutical composition according to claim 1, wherein an X-ray powder diffraction pattern of the crystal has a peak at least one diffraction angle (2θ) selected from the group consisting of 4.9°, 9.4°, 9.9°, 15.2°, 15.8°, 18.9° and 22.6° (±0.2°).

3. The pharmaceutical composition according to claim 1 or 2, wherein an X-ray powder diffraction pattern of the crystal has a peak at diffraction angles (2θ) of 4.9°, 9.4°, 9.9°, 15.2°, 15.8°, 18.9° and 22.6° (±0.2°).

4. The pharmaceutical composition according to any one of claims 1 to 3, further comprising an excipient and a disintegrant.

5. The pharmaceutical composition according to any one of claims 1 to 4, wherein the content of the lubricant is 0.5 wt% or more based on a total of the pharmaceutical composition.

6. The pharmaceutical composition according to any one of claims 1 to 5, wherein the lubricant is at least one selected from the group consisting of sodium stearyl fumarate, zinc stearate, aluminum stearate, calcium stearate, magnesium stearate, talc, and a sucrose fatty acid ester.

7. The pharmaceutical composition according to any one of claims 1 to 6, wherein the lubricant is sodium stearyl fumarate.

8. The pharmaceutical composition according to any one of claims 4 to 7, wherein the content of the excipient is 25 wt% or more based on the total of the pharmaceutical composition.

9. The pharmaceutical composition according to any one of claims 4 to 8, wherein the excipient is at least one selected from the group consisting of mannitol, lactose hydrate, fructose, glucose, sorbitol, corn starch, potato starch, wheat starch, and rice starch, preferably mannitol.

10. The pharmaceutical composition according to any one of claims 4 to 9, wherein the content of the disintegrant is 10 wt% or more based on the total of the pharmaceutical composition.

11. The pharmaceutical composition according to any one of claims 4 to 10, wherein the disintegrant is at least one selected from the group consisting of croscarmellose sodium, carmellose sodium, hydroxypropyl cellulose, carmellose, carmellose calcium, methylcellulose, crystalline cellulose, sodium lauryl sulfate, povidone, and polysorbate, preferably croscarmellose sodium or carmellose calcium.

12. The pharmaceutical composition according to any one of claims 1 to 11, wherein a content of the type 1 crystal of the p-toluenesulfonate salt of the compound represented by formula 1 is in a range of 5 wt% or more and 50 wt% or less based on the total of the pharmaceutical composition.

13. The pharmaceutical composition according to any one of claims 1 to 12, wherein a volume-based particle size of the type 1 crystal of the p-toluenesulfonate of the compound represented by formula 1 has d₁₀ of less than 5.76 µm, d₅₀ of less than 8.81 µm, or d₉₀ of less than 13.08 µm.

14. The pharmaceutical composition according to any one of claims 1 to 13, wherein the pharmaceutical composition is a powder containing the type 1 crystal of the p-toluenesulfonate salt of the compound represented by formula 1 as an active ingredient, and further containing mannitol, croscarmellose sodium, and sodium stearyl fumarate.

15. A pharmaceutical formulation comprising the pharmaceutical composition according to any one of claims 1 to 14, wherein the dosage form of the pharmaceutical formulation is selected from the group consisting of a powdered formulation, a powder, a granule, a tablet, and a capsule.
